# EUROPEAN PATENT APPLICATION

(11) **EP 2 248 889 A1**
(43) Date of publication of application: **10.11.2010**
(21) Application number: 09290335.0
(22) Date of filing: 06.05.2009
(51) Int. Cl.: C12N 5/10

(54) **Reversibly immortalized cells as well as methods relating hetero**

(71) Applicant: Sanofi-Aventis, 75013 Paris (FR)
(72) Inventor: The designation of the inventor has not yet been filed
(74) Representative: Schneider, Rudolf

(57) **Abstract**

The present invention relates to methods of producing a reversibly immortalized cell, cells obtainable by the above method, eukaryotic cells comprising one or more nucleic acid(s) coding for one or more immortalizing protein(s) and methods of re-differentiating these cells.

## Description

The present invention relates to methods of producing a reversibly immortalized cell, cells obtainable by the above method, eukaryotic cells comprising one or more nucleic acid(s) coding for one or more immortalizing protein(s) and methods of re-differentiating these cells.

As early as in the 19^{th} century a method for maintaining the beating of an isolated animal heart outside of the body was presented. At the beginning of the 20^{th} century experiments establishing the methodology of tissue culture were reported. Cell culture techniques were advanced significantly in the 1940s and 1950s to support research in virology. Animal cell culture became a common laboratory technique in the mid-1900's.

In general, the concept of cell culture involves the isolation of cells, also known as primary cells, from a tissue for *ex vivo* culture. For this, primary cells may be released by enzymatic digestion with enzymes such as collagenase, trypsin or pronase. Alternatively, pieces of tissue can be placed in suitable growth media and cells for growing are available for culture.

With the exception of cells derived from several tumors, most primary cell cultures have a limited lifespan. Furthermore, in many cases, primary cells have a limited proliferation potential in culture and undergo replication senescence with extended passages (i.e. endothelial cell, smooth muscle cells) or do not proliferate at all (e.g. hepatocytes, cardiomyocytes).

Therefore, cell-based experiments require multiple donors and in case of human cells create significant problems: donors' genetic variability, drug abuse etc.

The majority of surveyed experiments are performed on cell lines derived from tumors. However, tumor-derived cells often loose differentiation and expression of the important tissue-specific genes is genetically unstable. Therefore, it would be desirable to provide new cells combining the advantages of primary cells and tumor-derived cell lines by avoiding their disadvantages.

One reason for the limited proliferation potential of primary cells is progressive telomere shortening occurring at each cell division which ultimately leads to cell senescence. Methods of modifying cells have been reported wherein SV40 T antigen has been expressed which allows the cell to progress through an additional number of cell divisions before a second, irreversible crisis occurs. A process, whereby a primary cell harvested *in vivo* is induced to grow infinitely *in vitro,* is referred to as cell immortalization.

Several studies demonstrated that human cells can be transformed in a stepwise process requiring the combined expression of a series of growth-promoting and anti-senescence genes. Thus, through proper genetic manipulation, it may be possible to induce controlled proliferation of cells in culture. Moreover, it was possible to establish immortalized human cell lines in culture. The expression of the human telomerase catalytic unit can prevent growth arrest and immortalize human fibroblasts and epithelia cells. However, only a limited number could be immortalized by expression of human telomerase catalytic unit alone. Most cell types require the expression of a second gene such as the gene encoding SV40 T antigen. Continuous proliferation of immortalized cells in culture downregulates the expression of tissue-specific genes when compared with their primary cell counterparts. It has been reported that the expression of human telomerase catalytic subunits in combination with two oncogenes (SV40 T oncoprotein and an oncogene allel of H-ras) results in direct tumorogenic conversion of normal human cells, such as epithelial and fibroblast cells. Furthermore, immortalization of primary human cells has been also reported if human telomerase catalytic units, alone or in combination with one or more other genes, are expressed in renal proximal tubulae epithelial cells. While this method for immortalizing proximal tubulae cells was working well with these cells, it did not work with several other human primary cells including HUVECs, hepatocytes, smooth muscle cells and mesenchymal stem cells. Therefore, it was an object of the present invention to provide cells and methods for their production, wherein the cells or their precursor may be derived from different cell types and/or may be propagated without showing replicative senescence but at the same time having attractive properties of primary cells such as stable genotype and their level of differentiation. The development of a novel cellular model is of primary importance for biological and medicinal research, particularly for compound profiling, phenotype screening, target identification and/or target validation.

Surprisingly, it has been found that human papilloma virus (HPV) E6/E7 was able to immortalize cells when expressed in several eukaryotic cells including epithelial cells, endothelial cells and hepatocates (see Examples).

Accordingly, in a first aspect the present invention relates to a method of producing a reversibly immortalized cell, the method comprising
a) providing a eukaryotic cell,
b) genetically modifying the cell of step a) to enable expression of one or more nucleic acid(s) coding for one or more immortalizing protein(s) in the cell; and
c) expressing the one or more nucleic acid(s) in the cell of step b),
   wherein the one or more immortalizing protein(s) include(s) human papillomavirus (HPV) E6/E7 or a functionally active variant thereof.

As detailed above, the limited proliferation potential of primary human cells currently precludes their use in many applications. Methods for immortalizing cells have been described; however, they suffer from the drawbacks detailed above. The present invention discloses novel methods of producing a reversibly immortalized cell by genetically modifying the cell and expressing HPV E6/E7, optionally in conjunction with one or more other genes have been developed to provide alternative methods and preferably to overcome these disadvantages.

In step a) of the methods of the present invention the eukaryotic cell may be provided in that it is used directly after having been obtained from a subject. Alternatively, the cell may be cultured before carrying out the genetic modification of step b). The cell may be also derived from a cell line. However, primary cells, i.e. fresh isolated cell, or cell having been cultured for a short period of time, i.e. at most 4 weeks, preferably at most 3 weeks, more preferably at least two weeks, still more preferably at most 7, 6, 5, 4, 3, 2 or 1 day, are preferred.

The term "eukaryotic cell" includes cells derived from animals, plants, fungi, and protists. These cells are organized into complex structures enclosed within membranes. Eukaryotic cells differ from prokaryotic cells in that they posses a nucleus. Many eukaryotic cells contain other membrane-bound organelles such as mitochondria, chloroplasts and Golgi bodies. Cell division in eukaryotes is different from organisms without a nucleus (prokaryotes). It involves separating the duplicated chromosomes, through movements directed by microtubules. There are two types of division processes. In mitosis, one cell divides to produce two genetically identical cells. In meiosis is required in sexual reproduction.

As detailed above, the cell provided in step a) is genetically modified in step b) to enable expression of one or more nucleic acid(s) coding for one or more immortalizing protein(s) in the cell, wherein the one or more immortalizing protein(s) include(s) human papillomavirus (HPV) E6/E7. Means for genetic modification, particularly the introduction of genes are well known to the skilled person.

Human papillomavirus (HPV) E6/E7 relates to key cancer-causing proteins expressed by the HPV. Among the strains of HPV known to cause physical changes associated with cancer and pre-cancerous lesions, three oncoproteins are recognized: E5, E6 and E7. Accordingly, the term HPV E6/E7 relates to a combination of the proteins either as individuals or a fusion protein.

The full length HPV E6 genes encode proteins of about 150 to 160 amino acids, which contain two domains including zinc binding Cys-X-X-Cys motifs. E6 is of particular interest because it appears to have multiple roles in the cell and to interact with many other proteins. E6 primarily causes cancer by associating with and thereby inactivating p53 or pRB proteins, which act as tumor suppressors. When tumor suppressor proteins are inactivated tumor growth proceeds uncontrolled. E6's interaction with p53 and pRB marks these proteins for degradation by ubiquitylation and ubiquitin ligase. E6 is proven to act on other cellular proteins, and to positively affect telomerase activity, thus inactivating one of the ways by which cells are normally prevented from dividing unchecked. Additionally, E6 can act as a transcriptional cofactor - specifically, a transcription activator - when interacting with the cellular transcription factor, E2F1/DP1.

E6 can also bind to PDZ-domains, which are often found in signalling proteins. Binding at these locations causes transformation of the DLG protein and disruption of its suppressor function. E6 proteins also interact with the MAGUK (membrane-associated guanylate kinase family) proteins, which are usually structural proteins and which are believed to be involved with DLG's suppression activity. When E6 complexes with the PDZ domains on the MAGI proteins, it distorts their shape and thereby impedes their function. Overall, the E6 protein serves to impede normal protein activity in such a way as to allow a cell to grow and multiply at the increased rate characteristic of cancer.

E7 proteins are about 100 amino acids in size and also contain 2 copies of Cys-X-X-Cys domains as well as E6. As an oncoprotein, E7 functions through forming complex with and promote proteolysis of hypophosphorylated pRB, the active form of retinoblastoma tumor suppressor gene product. Formation of E7/pRB complex interfere the complex formation of pRB with E2F. So E2F is released from inactivated combination with pRB and continues its work as a transcription activator to promote DNA synthesis and cell proliferation.

In high risk HPV infected cells, E6 and E7 are usually co-expressed. The high-risk HPV E7 oncoproteins bind and degrade the pRB and the related pRB family members p107 and p130.17, as well as inactivate the cyclin-dependent kinase (CDK) inhibitors As a result, there comes aberrantly increased expression of cyclin E, cyclin A and aberrant CDK2 activity mediated by E2F. E2F can also activate p14ARF to stabilize p53. As cooperation with E7 to promote cell proliferation, the high-risk HPV E6 oncoprotein induces the rapid proteasomal degradation of p53 by interacting with E6-AP, a host cell protein.

The nucleic acid or amino acid sequence of E6 or E7 may be derived from any HPV type comprising or expressing the same. More than 100 different HPV types have been identified and are referred to by number. Types 16, 18, 31, 33, 35, 39, 45, 51, 52, 56, 58, 59, 68, 73 and 68 are examples of "high-risk" HPVs (because they can lead to cancer) and are therefore preferred sources for the above sequences. A highly preferred cDNA for HPV E6/E7 is provided as SEQ ID NO: 4.

The present invention also encompasses functionally active variants of HPV E6/E7. The variant could be an E6/E7 fragment, wherein the fragment is still capable of immortalizing a cell. This may include E6/E7 proteins with short C- and/or N-terminal deletions (e.g. deletions of at most 20, 19, 18, 17, 16, 15, 14, 13, 12, 11, 10, 9, 8, 7, 6 5, 4, 3, 2, or 1 amino acid). Alternatively or additionally, the E6/E7 protein may comprise one or more amino acid substitution(s). However, conservative amino acid substitutions, wherein an amino acid is substituted with a chemically related amino acid are preferred. Typical conservative substitutions are among the aliphatic amino acids, are among the amino acids having aliphatic hydroxyl side chains, are among the amino acids having acidic residues, among the amid derivates, among the amino acids with basic residues, or the amino acids having aromatic residues. The E6/E7 varient is functionally active according to the present invention, if it is still capable of immortalizing a cell, optionally in combination with other immortalizing proteins. The ability to immortalize a cell can be tested as described in the context of propagating the cell and in the Examples of the present invention, such as comparing the amount immortalized and control cells by means of immunostaining, determining total RNA, determining cell morphology, cell survival (optionally under disadvantegous conditions) or cell number. In the context of the present invention a variant has the ability to immortalize a cell, if the activity of the variant amounts to at least 10 %, preferably at least 25 %, more preferably at least 50 %, even more preferably at least 70 %, still more preferably at least 80 %, especially at least 90 %, particulary at least 95 %, most preferably at least 99 % of the activity of the E6/E7 protein without sequence alterations.

In the following description of the invention all details given with respect to E6/E7 also relate to functionally active variants thereof, unless stated otherwise.

However, most preferably, E6/E7 is a naturally occurring E6/E7, still more preferably HPV E6/E7, most preferably HPV E6/E7 coded by the cDNA is provided as SEQ ID NO: 4.

The term, "nucleic acid", refers to any nucleic acid molecule, preferably DNA, as discussed in detail herein. The nucleic acid molecule may be derived from a variety of sources, including DNA, cDNA, synthetic DNA, RNA or combinations thereof. Such nucleic acid sequences may comprise genomic DNA which may or may not include naturally occurring introns. Moreover, such genomic DNA may be obtained in association with promoter regions, poly A sequences or other associated sequences. Genomic DNA may be extracted and purified from suitable cells by means well known in the art. Alternatively, messenger RNA (mRNA) can be isolated from cells and used to produce cDNA by reverse transcription or other means.

Before the genetic modification usually a gene of interest, here one or more nucleic acid(s) coding for one or more immortalizing protein(s) in the cell, is/are isolated. Thereafter, the nucleic acid, preferably DNA or cDNA, is formulated in a manner to allow for modification of the cell. There are various methods of introducing foreign DNA into a eukaryotic cell, e.g. by transfection, i.e. by a process of introducing nucleic acids into cells by non-viral methods, or by transduction, i.e. genetic alterations of a cell resulting from introduction of genetic material by a virus.

Many materials have been used as carriers for transfection, which can be divided into three kinds: (cationic) polymers, liposomes and nanoparticles. This may be accomplished by transfection by, e.g. calcium phosphate precipitation. Another method is the use of cationic polymers such as DEAE-dextran or polyethylenimine. The negatively charged DNA binds to the polycation and the complex is taken up by the cell via endocytosis. A direct approach to transfection is the gene gun, where the DNA is coupled to a nanoparticle of an inert solid (commonly gold) which is then "shot" directly into the target cell's nucleus.

For transfection, a gene or cDNA may be transferred into a suitable vector. The vector may be a plasmid or a viral vector. The nucleic acid may be introduced into the vector by the use of restriction enzymes and ligases as known to the skilled person. Once the vector is obtained, it can be used to transform the cell of interest.

After transformation, the genetically modified cell may be selected from those that have failed to take up the vector in various ways. One method is screening with DNA probes that can stick to the gene of interest that was supposed to have been transplanted. Another is the use of markers. Marker genes may be utilized to assay for the presence of the vector, and thus, to confirm infection and integration. The presence of a marker gene ensures the selection and growth of only those host cells which express the inserts. Typical selection genes encode proteins that confer resistance to antibiotics and other toxic substances, e.g., histidinol, puromycin, hygromycin, neomycin, methotrexate, and cell surface markers. Alternatively, genes coding for detectable proteins may be used as markers, such as fluorescent proteins including green fluorescent protein (GFP) or yellow fluorescent protein (YFP).

Furthermore, the gene or cDNA may be combined with suitable elements allowing for efficient transfer of the nucleic acid or expression of the same. These may be elements regulating transcription of the nucleic acids such promoter sequences, transcription initiation sequences, enhancer sequences, selectable elements, and reporter genes. Additionally, suitable restrictions sites may be added to the nucleic acid of interest. Furthermore, elements regulating translation by capping, splicing, addition of a Poly(A) Tail and altering the sequence-specific nuclear export rates may be present.

If a vector is used, the one or more nucleic acids may be part of one or more vectors. If more than one vector is used they may be introduced into the cell either simultaneously or sequentially. Viral vectors are a tool commonly used to deliver genetic material into cells. This process can be performed inside a living organism (*in vivo*) or in cell culture (*in vitro*). Viruses have evolved specialized molecular mechanisms to efficiently transport their genomes inside the cells they infect. Delivery of genes by a virus is termed transduction and the infected cells are described as transduced.

Viral vectors were originally developed as an alternative to transfection of naked DNA for molecular genetics experiments. Protein coding genes can be expressed using viral vectors, commonly to study the function of the particular protein. Viral vectors, especially retroviruses, are widely used to transfer genetic material into a cell (see also below). Other types of viral vectors are adenoviruses and Adeno-associated viruses (AAV). AAV can infect both dividing and non-dividing cells and may incorporate its genome into that of the host cell. These features make AAV a very attractive candidate for creating viral vectors. The sequence of a highly preferred vector is given as SEQ ID NO: 6.

After the having the cell genetically modifying in step b) the one or more nucleic acid(s) coding for one or more immortalizing protein(s) in the cell are expressed. The cell may express the one or more nucleic acid(s) coding for one or more immortalizing protein(s) without any further interaction, e.g. if the expression is regulated by a constitutively active promoter. Alternately, the expression may need to be induced, e.g. if an inducible promoter is linked to the one or more nucleic acid(s).

In this method, a eukaryotic cell, preferably a primary cell as defined below, is provided and genetically modified so that the modified cell expresses one or more nucleic acid(s) coding for the immortalizing protein(s) human papillomavirus (HPV) E6/E7. In addition the one or more nucleic acid(s) may also code for further immortalizing proteins including SV40 Large T antigen (TAG); telomerase catalytic subunit (TERT); or SV40 Large T antigen (TAG) and telomerase catalytic subunit (TERT). Accordingly, by means of genetically modification the nucleic acid(s) coding for HPV E6/E7 may be the only nucleic acids introduced into the cell.

However, in a preferred embodiment of the invention the one or more immortalizing protein(s) comprise or consist of:
- HPV E6/E7 and SV40 Large T antigen (TAG); or
- HPV E6/E7 and telomerase catalytic subunit (TERT); or
- HPV E6/E7 and SV40 Large T antigen (TAG) and telomerase catalytic subunit (TERT).
Functionally active variants of these are also included.

SV40 large T antigen (Simian Vacuolating Virus 40 Tag, also referred to as TAG) is a hexamer protein that is an oncogene derived from the polyomavirus SV40 which is capable of transforming a variety of cell types. The transforming activity of TAG is due in large part to its perturbation of the retinoblastoma (pRB) and p53 tumor suppressor proteins. In addition, TAG binds to several other cellular factors, including the transcriptional co-activators p300 and CBP, which may contribute to its transformation function.

TAG is a product of an early gene transcribed during viral infection by SV40, and is involved in viral genome replication and regulation of host cell cycle. SV40 is a double-stranded DNA virus, belongs to Papovavirus family, Polyomavirus genus. Polyomaviruses infect a wide variety of vertebrates and it caused solid tumours at multiple sites. SV40 DNA replication is initiated by binding of TAG to the origin region of the genome. The function of TAG is controlled by phosphorylation, which attenuates the binding to the SV40 origin. Protein-protein interactions between TAG and DNA polymerase-alpha directly stimulate replication of the virus genome. TAG also binds and inactivates tumor suppressor proteins (p53, p105). This causes the cells to leave G1 phase and enter into S phase, which promotes DNA replication.

The term "telomerase catalytic subunit" or "TERT" as used herein refers to a polypeptide sequence possessing telomerase catalytic activity. Telomerase is an enzyme that adds specific DNA sequence repeats ("TTAGGG" in all vertebrates) to the 3' ("three prime") end of DNA strands in the telomere regions, which are found at the ends of eukaryotic chromosomes. The telomeres contain condensed DNA material, giving stability to the chromosomes. The enzyme is a reverse transcriptase that carries its own RNA molecule, which is used as a template when it elongates telomeres, which are shortened after each replication cycle. It consists of two molecules each of telomerase catalytic subunit also referred to as Telomerase Reverse Transcriptase (TERT); Telomerase RNA (hTR or TERC); and dyskerin. TERT is a reverse transcriptase, which creates single-stranded DNA using single-stranded RNA as a template.

In the context of the present invention also functionally active variants of TAG and/or TERT may be used. The terms "variant" and "functionally active" are as defined above in the context of the variant of E6/E7. In the following description of the invention all details given with respect to E6/E7 also relate to functionally active variants thereof, unless stated otherwise.

In one example the first vector contains a polynucleotide encoding HPV E6/E7, while the second vector contains a nucleic acid coding for polynucleotide coding for SV40 Large T antigen (TAG); telomerase catalytic subunit (TERT); or SV40 Large T antigen (TAG) and telomerase catalytic subunit (TERT).

In another aspect the present invention relates to method of producing a reversibly immortalized cell, the method comprising
a) providing a eukaryotic cell,
b) genetically modifying the cell of step a) to enable expression of one or more nucleic acid(s) coding for one or more immortalizing protein(s) in the cell; and
c) expressing the one or more nucleic acid(s) in the cell of step b),
   wherein the at least one of the one or more immortalizing protein(s) is selected from the group consisting of human papillomavirus (HPV) E6/E7, SV40 Large T antigen and telomerase catalytic subunit (TERT), or a functionally active variant of any of these, and wherein expression of one or more of the nucleic acid(s) coding for one or more immortalizing proteins is controlled by an EF1alpha promoter.

Surprisingly, it has been found that promoter EF1alpha (for details see below) provides a better level of expression than other. Several promoters have been tested in different primary cells. It could be shown by expressing GFP (green fluorescent protein) cDNA under the control of different promoters that EF1alpha promoter moderate provided a expression level in contrast to CMV (Cytomegalovirus) promoter and MSCV (Mouse Cytomegalovirus) promoter, which led to extremely high or extremely low levels of expression, respectively. All terms of this aspect of the invention are defined as detailed in this description.

In a further embodiment of the present invention the methods of the present invention further comprise
d) propagating the cell of step c).

According to the present invention the cells of step c) may be propagated the cells under conditions effective to allow cell viability, proliferation and integrity. Herein, propagating is by culturing *in vitro.* Propagating refers to the increase of the number of cells as a result of cell growth and division, which may be achieved by means of cell culture. Preferably, it is is at least one cell doubling. Possible methods for monitoring proliferation include, but are not limited to, detection of an antigen associated with proliferation, measurement of DNA synthesis, and detection of reduction of the intracellular environment. DNA synthesis may be measured by, for instance, quantitating ³H-thymidine incorporation or 5-bromodeoxyuridine (BrdU) incorporation. Reduction of the intracellular environment may be monitored by tetrazolium salt reduction. Viability refers to the ability of a cell or cells to survive and reproduce. Commonly used assays for measuring cell viability include, but are not limited to, Trypan Blue exclusion, Neutral Red staining, crystal violet inclusion, and ⁵¹ Cr release.

Once immortalized, stable clones generated by this method can be cultured for at least two months in culture. In a preferred embodiment these stable clones can be passaged in culture for at least six months, preferably at least one year, and most preferably at least two years. These stable clones continue to possess high levels of the functions and characteristics associated with the cell type from which they are derived.

For cell culture, cells are grown under suitable conditions conducive to the production of cells. Aside from temperature and gas mixture, the most commonly varied factor in cell culture systems is the growth medium. Recipes for growth media can vary in pH, glucose concentration, growth factor and the presence of other nutrient components among others. Growth factors used for supplement media are often derived from animal blood such as calf serum. Culture conditions vary widely for each cell type, and variation of conditions for a particular cell type can result in different phenotypes being expressed.

Cells can be grown in suspension or adherent cultures. Some cells naturally live in suspension, without being attached to a surface, such as cells that exist in the bloodstream. There are also cells that have been modified to be able to survive in suspension cultures so that they can be grown to a higher density than adherent conditions would allow. Adherent cells require a surface, such as tissue culture plastic, which may be coated with extracellular matrix components to increase adhesion properties and provide other signals needed for growth and differentiation. Most cells derived from solid tissues are adherent. Another type of adherent culture is organotypic culture which involves growing cells in a three-dimensional environment as opposed to two-dimensional culture dishes. This 3D culture system is biochemically and physiologically more similar to in vivo tissue, but is technically challenging to maintain because of many factors (e.g. diffusion). Suitable methods and their selection are known to the skilled person. Furthermore, suitable methods fort he cells immortalized are also given in the Examples.

In a preferred embodiment, the one or more nucleic acid(s) coding for immortalizing protein(s) is/are introduced by using one or more retroviral vector(s), particularly one or more lentiviral vector(s).

Retroviruses are the one of mainstays of current gene transfer approaches. The recombinant retroviruses such as the Moloney murine leukemia virus have the ability to integrate into the host genome in a stable fashion. Accordingly, in a preferred embodiment the one the one or more nucleic acid(s) coding for immortalizing protein(s) is/are introduced by using one or more retroviral vector(s), particularly one or more lentiviral vector(s).

A retrovirus is a virus with an RNA genome that replicates by using a viral reverse transcriptase enzyme to transcribe its RNA into DNA in the host cell. The DNA is then incorporated into the host's genome by an integrase enzyme. The virus thereafter replicates as part of the host cell's DNA. Retroviruses are enveloped viruses that belong to the viral family Retroviridae. The virus itself stores its nucleic acid, in the form of mRNA (including the 5'cap and 3'PolyA inside the virion) genome and serves as a means of delivery of that genome into cells it targets as an obligate parasite, and constitutes the infection. Once in the host's cell, the RNA strands undergo reverse transcription in the cytosol and are integrated into the host's genome, at which point the retroviral DNA is referred to as a provirus. In general retrovirus are specified as exogenous (including the following genera Alpharetrovirus such as Avian leukosis virus; Betaretrovirus such as Mouse mammary tumour virus; Gammaretrovirus such as Murine leukemia virus; Deltaretrovirus such as Bovine leukemia virus; Human T-lymphotropic virus; Epsilonretrovirus such as Walleye dermal sarcoma virus; Lentivirus such as Human immunodeficiency virus 1 as well as Simian and Feline immunodeficiency viruses; and Spumavirussuch as Chimpanzee foamy virus) and endogenous (including Class I, which are most similar to the gammaretroviruses, Class II, which are most similar to the betaretroviruses and alpharetroviruses, and Class III, which are most similar to the spumaviruses).

Lentivirus is a genus of slow viruses of the Retroviridae family, in general characterized by a long incubation period. Lentiviruses can deliver a significant amount of genetic information into the DNA of the host cell, so they are one of the most efficient methods of a gene delivery vector. Lentivirus is primarily a research tool used to introduce a gene product into in vitro systems or animal models. Lentiviral infection have advantages over other gene-therapy methods including high-efficiency infection of dividing and non-dividing cells, long-term stable expression of a transgene, and low immunogenicity. Lentiviruses are a subclass of Retroviruses. They have recently been adapted as gene delivery vehicles (vectors) thanks to their ability to integrate into the genome of non-dividing cells, which is the unique feature of Lentiviruses as other Retroviruses usually can infect only dividing cells. The viral genome in the form of RNA is reverse-transcribed when the virus enters the cell to produce DNA, which is then inserted into the genome at a random position by the viral integrase enzyme. Therefore, in a preferred embodiment of the present invention the one or more nucleic acid(s) coding for immortalizing protein(s) is/are introduced by using one or more lentiviral vector(s). Five serogroups of lentiviruses are recognized, reflecting the vertebrate hosts with which they are associated (primates, sheep and goats, horses, cats, and cattle). HIV, SIV, and FIV are all examples of lentiviruses. Although transmission is generally via infectious particles, lentiviruses are capable of infecting neighboring cells in direct contact with the host cells, without having to form extracellular particles.

In a preferred embodiment, the eukaryotic cell in the context of the present invention is an animal cell, more preferably a chordate cell, still more preferably a vertebrate, even more preferably a mammalian cell, particularly a human, primate or rodent cell. Additionally, or alternatively, the eukaryotic cell may be a primary cell or non-primary cell. However, primary cells, i.e. cells obtained from a subject directly before use in the present method, are preferred for use in the methods of producing a reversibly immortalized cell. The term "primary cell" is as defined above.

Furthermore, the eukaryotic cell may be any cell type. However, somatic cells, i.e. any cells forming the body of an organism, as opposed to germline cells, are preferred. In mammals, germline cells are the spermatozoa and ova. Every other cell type in the mammalian body is a somatic cell. Examples of somatic cells include those derived from internal organs, skin, bones, blood, and connective tissue. Preferably, the cell is differentiated cell, i.e. a cell of a specialized cell type expressing a particular subset of all the genes. Preferred differentiated cells include an endothelial cell, a cardiomyocyte, a smooth muscle cell, a hepatic cell, a podocyte or a kidney cell.

In another embodiment of the present invention, the expression of one or more of the nucleic acid(s) coding for one or more immortalizing proteins is controlled by an EF1alpha promoter.

A promoter is a region of DNA that facilitates the transcription of a particular gene. Promoters are typically located near the genes they regulate, on the same strand and upstream (towards the 5' region of the sense strand). The promoter is usuable chosen in accordance with the cell, in which expression of the gene/DNA is intended. Examples of suitable promoters include for example, EF1alpha, PGK, the Moloney murine leukemia virus promoter-enhancer element, the human cytomegalovirus enhancer, the vaccinia P7.5 promoter or the like, however EF1alpha promoter is preferred. EF1alpha promoter is particularly suitable for lentiviral vectors and expression in eukaryotic cells. The promoter may be eukaryotioc EF1alpha promoter or preferably human EF1alpha promoter. The sequence of a suitable promoter is given as SEQ ID NO: 1.

In another embodiment of the present invention, one or more of the nucleic acid(s) coding for one or more immortalizing proteins is/are flanked by loxP (locus of X-over P1) sites, which allows for removal of the one or more of the nucleic acid(s) in the presence of Cre or a Cre variant.

Cre, a 38-kDa recombinase from bacteriophage P1, utilizes its endonuclease activity to catalyze recombination between two identical loxP sites. The enzyme Cre requires no accessory proteins or cofactors and functions efficiently in vitro and under a wide variety of cellular conditions. The recombination site recognized by Cre is a double-stranded DNA sequence known as loxP. Each loxP site consists of two inverted repeats separated by an asymmetrical core region. Cre binds to the inverted repeats and cleaves the DNA in the core region to facilitate DNA strand exchange reactions. The original loxP sequence consists of 34 bp. There exists an asymmetric 8 bp sequence in between with two sets of palindromic, 13 bp sequences flanking it. The detailed structure is given below.

13bp 8bp 13bp
ATAACTTCGTATA - GCATACAT -TATACGAAGTTAT (SEQ ID NO: 7)

Preferred loxP sites are given as SEQ IDNO: 2 and 3.

The Cre (Cyclization Recombination) protein consists of 4 subunits and two domains: The larger carboxyl (C-terminal) domain, and smaller amino (N-terminal) domain. The total protein has 343 amino acids. The C domain is similar in structure to the domain in the Integrase family of enzymes isolated from lambda phage. This is also the catalytic site of the enzyme. A Cre variant polypeptide may differ from Cre with regards to specific amino acid sequence, but will maintain the recombinase activity of Cre. Generally, differences are limited so that the sequences of the reference polypeptide and the variant are closely similar overall and, in many regions, identical. A variant and reference polypeptide may differ in amino acid sequence by one or more substitutions, additions, or deletions in any combination. A substituted or inserted amino acid residue may or may not be one encoded by the genetic code. A variant of a polypeptide may be naturally occurring or it may be a variant that is not known to occur naturally.

The Cre/lox system is used as a genetic tool to control site specific recombination events in genomic DNA. This system has allowed researchers to manipulate a variety of genetically modified organisms to control gene expression, delete undesired DNA sequences and modify chromosome architecture. The system involves the Cre, by which double stranded DNA is cut at both *loxP* sites by the Cre and then ligated back together. The efficiency of recombination depends on the orientation of the *loxP* sites. For two lox sites on the same chromosome arm, inverted *loxP* sites will cause an inversion, while a direct repeat of *loxP* sites will cause a deletion event. If *loxP* sites are on different chromosomes it is possible for translocation events to be catalysed by Cre induced recombination.

In another aspect the present invention provides a cell obtainable by the method of the invention, or a progeny thereof.

In still another aspect the present invention provides a eukaryotic cell comprising one or more nucleic acid(s) coding for one or more immortalizing protein(s), wherein at least one of the one or more immortalizing protein(s) is selected from the group consisting of human papillomavirus (HPV) E6/E7, SV40 Large T antigen and telomerase catalytic subunit (TERT) and wherein expression of one or more of the nucleic acid(s) coding for one or more immortalizing protein(s) is under the control of an EF1alpha promoter.

The cell may be further characterized as detailed above in the context of the method of producing a reversibly immortalized cell, according to the invention. Particularly, the cell or progeny thereof may be characterized by one or more of the following properties:
i) the one or more nucleic acid(s) coding for immortalizing protein(s) is/are located on one or more retroviral vector(s), particularly one or more lentiviral vector(s);
ii) the cell comprises a further genetic modification;
iii) the cell is a mammalian cell, particularly a human, primate or rodent cell;
iv) the cell is derived from a primary cell;
v) the cell is derived from a differentiated cell, particularly an endothelial cell, a cardiomyocyte, a smooth muscle cell, a hepatic cell, a podocyte or a kidney cell;
vi) expression of the nucleic acid(s) coding for the one or more immortalizing protein(s) is under the control of an EF1alpha promoter; and/or
vii) one or more of the nucleic acid(s) coding for one or more immortalizing protein(s) is/are flanked by loxP (locus of X-over P1) sites.

In another aspect the present invention provides a method of re-differentiating the cell of the present invention, or progeny thereof, the method comprising preventing expression of the one or more nucleic acid(s) coding for one or more immortalizing protein(s).

The use of immortalized cells provides a means to overcome the shortage of primary cells available for such procedures. Immortalized cells in which immortalization has been reversed exhibit decreased growth and proliferation. After 2-3 weeks, the cells develop morphology consistent with cell senescence, suggesting that regulation of the cell cycle remains intact in the immortalized cells. In addition, cells in which immortalization has been reversed exhibit increased differentiation functions. Cells in which immortalization has been reversed may be used as source for biological and medicinal research, particularly for compound profiling, phenotype screening, target identification and/or target validation. For this, cells may be first immortalized and cultured (and propagated) until re-differentiated cells are needed. This ensured the use of a economic and homogenous cell source for many experiments and over a long period of time, which is of cause desirable.

Re-differentiating the cell may be accomplished by any suitable method of preventing expression of a nucleic acid. This may be done by antisense nucleic acids binding to the respective mRNA thus preventing translation of the same. Alternatively controllable promoters, such as inducible promoters, may be used. As their name says, the activity of these promoters is induced by the presence or absence of biotic or abiotic factors. Inducible promoters are a very powerful tool in genetic engineering because the expression of genes operably linked to them can be turned on or off at certain stages of development of an organism or in a particular tissue. Inducible promoters are grouped chemically-regulated promoters, including promoters whose transcriptional activity is regulated by the presence or absence of alcohol, tetracycline, steroids, metal and other compounds; and physically-regulated promoters, including promoters whose transcriptional activity is regulated by the presence or absence of light and low or high temperatures.

In a preferred embodiment of the method of re-differentiating the cell, one or more of the nucleic acid(s) coding for one or more immortalizing proteins is/are flanked by loxP (locus of X-over P1) sites and the preventing is effected by Cre recombinase or a functionally active Cre variant.

Preferably, if loxP sites are used, the immortalization process can be reversed by introducing a vector containing a polynucleotide encoding Cre or a Cre variant into the cells. When the one or more nucleic acid(s) coding for one or more immortalizing protein(s) are removed from the cells by Cre-mediated recombination, the cell type-specific activities of the respective cell or clone are generally upregulated. Removal of the genes responsible for cell immortalization also significantly decreases the rate of cell proliferation, and ultimately leads to cell senescence.

In another preferred embodiment of the method of re-differentiating the cell, a Cre recombinase gene or a functionally active Cre variant gene is introduced into the cell using a retroviral vector, particularly a lentiviral vector. Vectors and methods of transferring nucleic acids are detailed above.

In another aspect the present invention provides a cell obtainable by the method of re-differentiating the cell, according to the present invention, or a progeny thereof.

The following Figures and Examples are intended to illustrate various embodiments of the invention. As such, the specific modifications discussed are not to be construed as limitations on the scope of the invention. It will be apparent to one skilled in the art that various equivalents, changes, and modifications may be made without departing from the scope of the invention, and it us understood that such equivalent embodiments are to be included herein.

### FIGURES

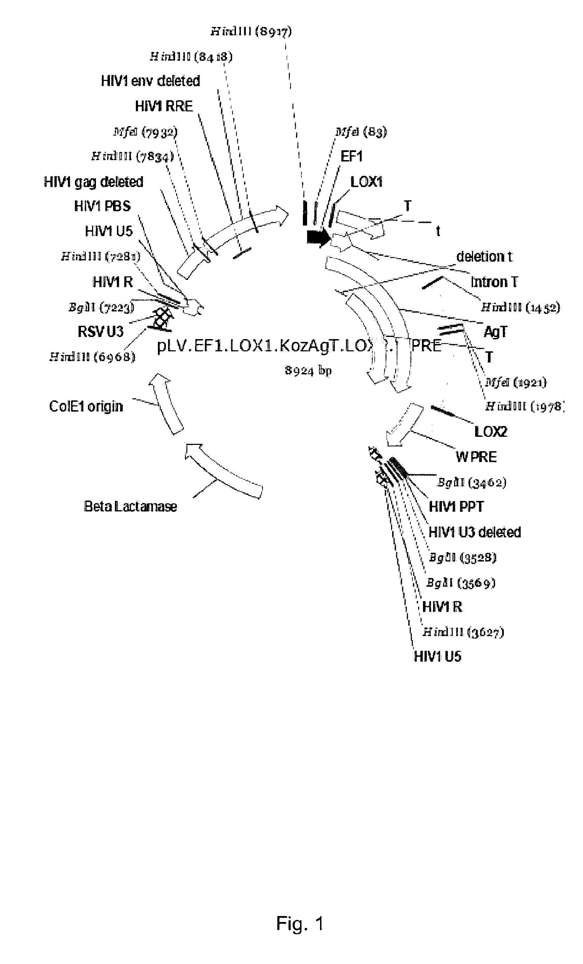

**Fig 1** shows a schematic illustration of the pLV-EF1-Lox1-AgT-Lox2 lentiviral vector of the VECTALYS System. cDNAs were constructed in a lentiviral plasmid under control of the EF1A promoter with the lox sites on both sides **(****Fig.1****),** which allow, when necessary, to excise the "immortogene".
EF1 = EF1alpha promotor
AgT = SV40 Large T Antigen
T = SV40 small t Antigen
LOX1 / LOX2 = Cre recombinase recognition site
WPRE = Woodchuck Post-transcriptional Regulatory Element

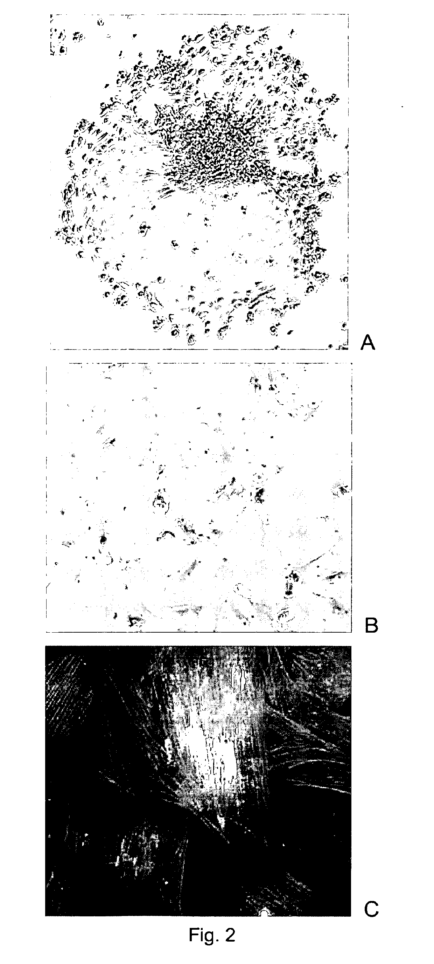

**Fig 2** shows expression of synaptopodin in a primary cultured neonatal rat podocyte cell line. **A:** Phase contrast morphology of undifferentiated primary podocytes. The cobblestone morphology of undifferentiated podocytes growing under standard conditions is shown. The cells form a monolayer as they reach confluence. **B:** Arborized podocytes maintained under standard conditions are very large and flat. **C:** Podocyte-specific protein synaptopodin starts to be expressed after 14 days at standard culture conditions (synaptopodin - light grey and actin stress fibres - dark grey)

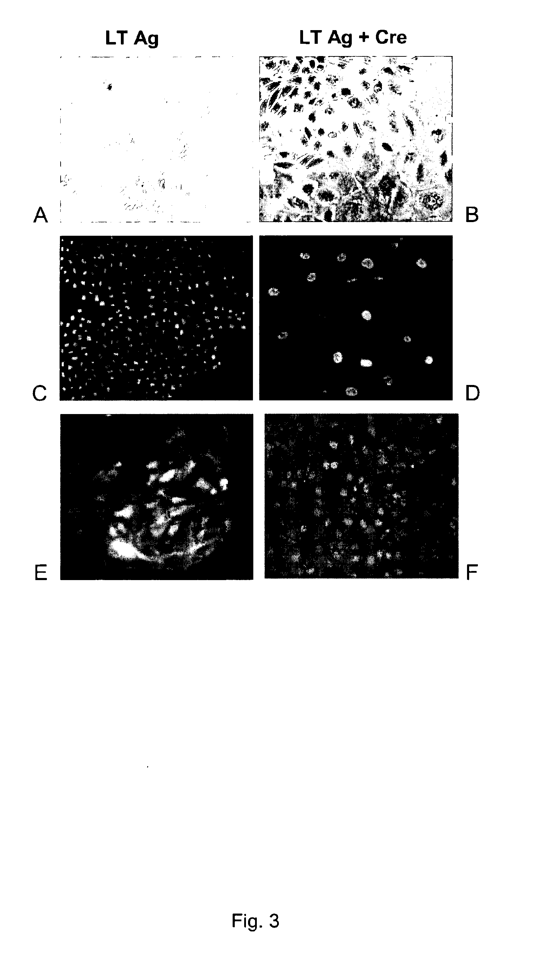

**Fig 3** shows that markers of differentiated podocytes re-appear after Cre-mediated excision of the "immortogene". **A:** (Phase contrast): **typical** cobblestone morphology of undifferentiated podocytes during active growth induced by LT Ag + E6/E7 overexpression. The cells form a monolayer as they reach confluence. **B:** Arborized podocytes after transduction of Crerecombinase. (large and flat). **C:** Nuclear Expression of LT Ag (grey) - 100% of cells is positive. **D:** After transduction with Cre recombinase many cells lose SV40 LT Ag (arrows).

### NOTE: Arrows seem to be missing

**E:** Synaptopodin and WT1 **(F)** are expressed in re-differentiated podocytes.

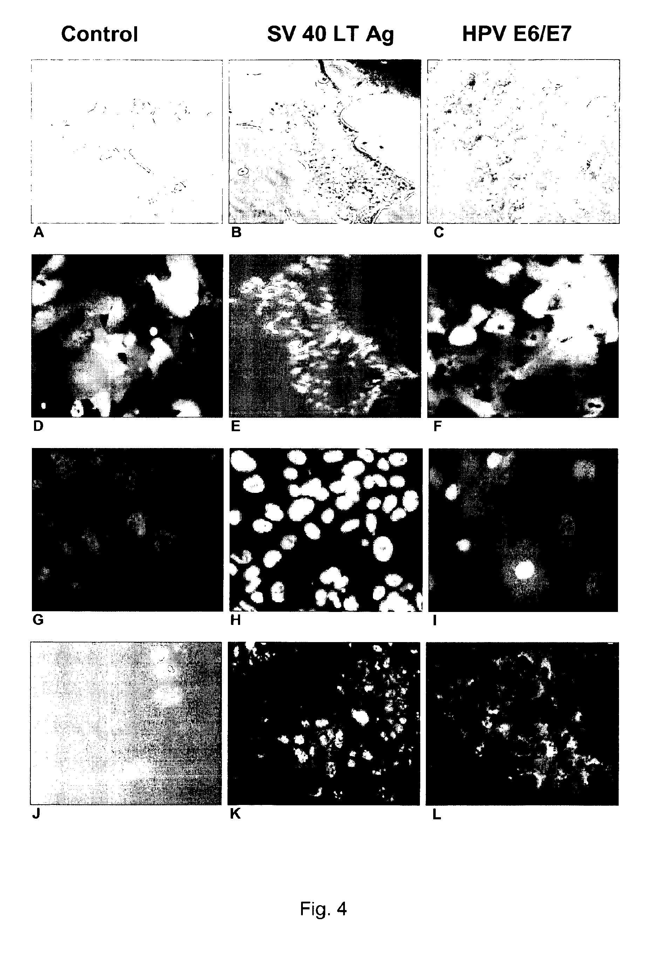

**Fig 4** shows an immunocytochemical comparison of the primary (control) and immortalized human hepatocytes. **A, B** and **C** - Light microscopy images; D, E and F -GFP fluorescence (each cell type was transduced with lentiviral GFP). Control hepatocytes are big polygonal cells **(A and D);** expression of SV40LTag transforms them into extremely small oval cells **(B and E);** after transduction with HPV E6E7 they have slightly reduced size in comparison with the control cells but are significantly bigger than SV40 LTag immortalized ones(compare F with D and E). **G-I:** immunostaining of **p53** (grey). **p53** basal level in control cells is very low **(G),** and in SV40 LTAg it accumulates in nuclei to extremely high level **(H);** in E6E7 immortalized cells its level is low in majority of cells **(I). J-L** immunostaining of CDC2, cyclin-dependent kinase 1 (CDK1); it can not be in primary hepatocytes as they do not proliferate **(J),** whereas in AgT immortalized cells **(K)** and also in E6E7 immortalized cells **(L)** CDC2 expression was significantly increased correlating with the increase of the CDC2 mRNA (see Table 2).

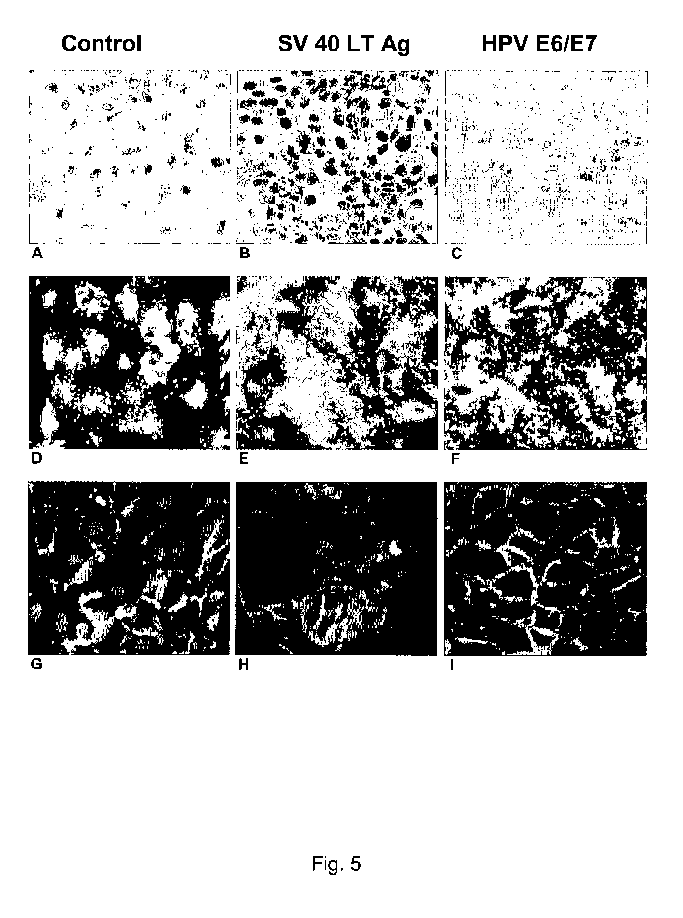

Fig 5 shows endothelial cell-specific markers continue to be expressed in the immortalized HUVECs. Freshly isolated HUVECs (left panel), AgT immortalized (middle panel) and E6E7 immortalized (right panel) HUVECs. Light microscopy images **(A, B** and **C).** Typical endothelial markers von Willebrand Factor **(D-F:** vWF, light grey) and PECAM (**G-I,** CD31, dark grey) are equally expressed in control **(D and G),** in AgT **(E and H)** and E6E7 **(F and I)** immortalized cells. Nuclei (dark grey) were stained with Hoechst 422.

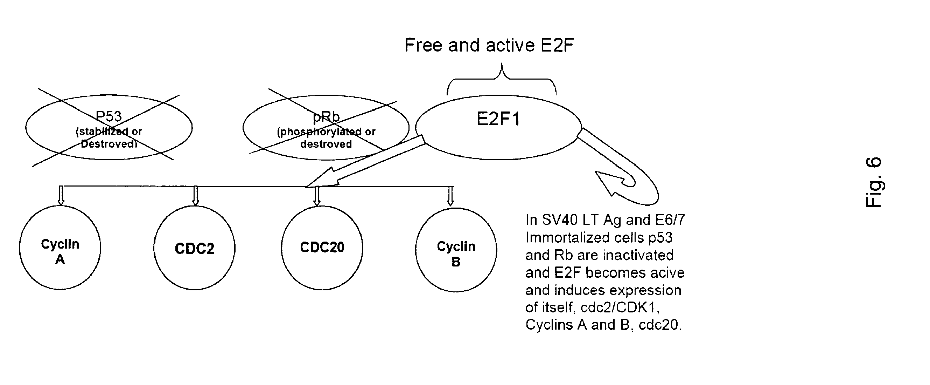

**Fig 6** shows putative mechanism of SV40 LT Ag- and E6/E7-induced proliferation. In non-proliferating cells the expression of cdc2/CDK1, Cyclins A and B, cdc20 is repressed by p53 and "repressor E2F". In SV40 LT Ag and E6/7 immortalized cells p53 and Rb are inactivated and E2F becomes active and induces expression of itself, cdc2/CDK1, Cyclins A and B, cdc20.

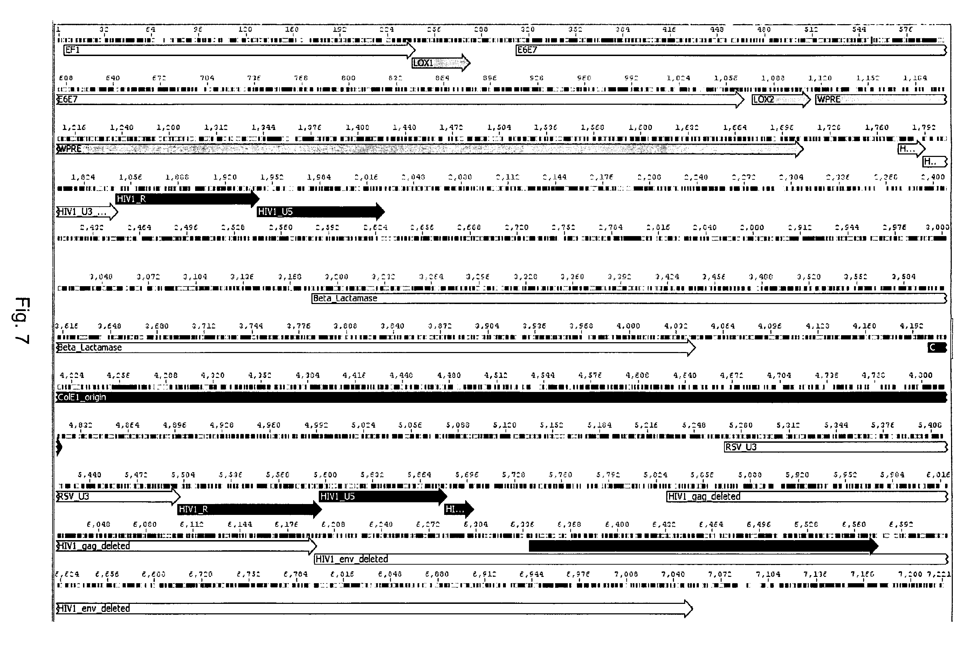

**Fig 7** shows pLV-EF1-LOX1-E6E7-LOX2 (Vectalys)

### EXAMPLES

### MATERIALS and METHODS

***Lentiviral constructs:*** All cDNAs were ordered from VECTALYS (France). Lentiviral particles were generated according to the standard protocols and had a titer of 1x10⁶-2x10⁶ TU/ml. An efficiency of infection has been measured using lentiviral GFP.

***Preparation of primary cells and culture conditions:*** To generate primary cultures of rat glomerular epithelial cells, glomeruli were isolated from neonatal rat kidneys. A good preparation is considered when 95% of the cellular constituents are glomeruli, with the remainder comprising tubules. After isolation glomeruli were cultured in DMEM with 10% FCS and 1% antibiotics on collagen-coated 24 well plates. 24 hours later podocytes precursors start to grow out from the attached glomeruli After 3 day culturing, outgrowing epithelial cells were trypsinized and passed through sieves with a 25-µm pore size to remove the remaining glomerular cores, which primarily consisted of mesangial and endothelial cells.

Primary human-derived endothelial cells can be routinely harvested from human umbilical cords. In brief, umbilical cord veins were rinsed of blood products with warm PBS, pH 7.4, and the vein was filled with warm collagenase A (Roche Diagnostics; used at a concentration of 320 U/ml in PBS). After 20 min of incubation in warm PBS, the digest was collected into centrifuge tubes containing heat-inactivated FCS (supplied by Gibco/Invitrogen Corp., Karlsruhe, Germany) to neutralize the collagenase activity, and cells were pelleted. The supernatant was discarded, and the pellet was resuspended in endothelial cell growth medium including FCS, ECGS/H, hEGF, hbFGF and HC-500 (Medium and SupplementPack from PromoCell) and cultured on collagen-I-coated T75 flasks and grown to confluence in a 37°C humidified incubator for 4-5 days. Cells were heavily seeded to minimize cell growth and thereby permit HUVEC to express CD14, which is otherwise lost. Consequently, only first passaged HUVEC were used for all experiments.

Primary human hepatocytes (PHH) were obtained from Cambrex (Weinheim, Germany) and cultured on collagen-I-coated dishes (Becton Dickinson Labware, San Jose, USA) in HBM basal hepatocyte media (Cambrex Bioproducts, Heidelberg, Germany), supplemented with HCM Single Quots (Cambrex Bioproducts, Heidelberg, Germany).

***Retroviral Construct and Virus Infection:*** Primary neonatal rat podocytes were isolated (as described below) and infected with single lentiviral construct containing a SV40 LT antigen (LT Ag) gene flanked by loxP sites or a vector containing human papillomavirus (HPV) E6 and E7 alone or in combination. Within first ten days, primary podocytes changed its phenotype and morphology and started to proliferate. Cells were splitted every 3 day and stable clones were isolated and frozen for further experiments. These stable clones morphologically resembled cells which initially grow out of glomeruli and not of differentiated podocytes. After infection with lentiviral vector containing a Cre recombinase cells ended proliferation and after 3-10 days changed their morphology to a specific for the differentiated podocytes.

The same immortalization procedure has been applied to HUVEC and human hepatocytes. HUVECs did not change their morphology despite high level of expression of LT Ag or E6/E7 but started active proliferation. Hepatocytes after infection with LT Ag or E6/E7 changed dramatically their phenotype and after introduction of Cre acquired their normal one.

***Antibodies:*** Primary antibodies: monoclonal WT-1 (F-6), CDC2 p34 (B-6), PCAM-1 (CD31), SV40 T Ag (Pab 108) and polyclonal vWF (C-20) (Santa Cruz Biotechnology, Heidelberg, Germany); polyclonal ZO-1 (Zymed, San Francisco, CA); monoclonal Synaptopodin (Progen, Heidelberg, Germany); polyclonal p53 (Cell Signalling). Secondary antibodies: Alexa Fluor® 488 goat anti-mouse IgG (H+L) *2 mg/mL*, Alexa Fluor® 594 goat anti-mouse IgG (H+L) *2 mg/mL*, Alexa Fluor® 488 goat anti-rabbit IgG (H+L) *2 mg/mL* and Alexa Fluor® 594 goat anti-rabbit IgG (H+L) *2 mg/mL* (Invitrogen, Karlsruhe, Germany).

***Immunostaining:*** Immunofluorescence staining of cells growing on collagen type I-coated chamber slides (VWR International GmbH, Darmstadt, Germany) was performed. The cells were fixed either with Methanol at -20°C for 5 min or with 4% formaldehyde in phosphate-buffered saline (PBS) for 5 min, followed by permeabilization with 0.3% Triton X-100 in PBS for 10-15 min at room temperature. After being rinsed with PBS, non-specific binding sites were blocked with SuperBlock (Blocking Buffer in PBS, Thermo Scientific, Braunschweig, Germany) with 0,2% Tween 20 (Serva, Amstetten, Austria) for at least 30 min. Primary antibodies prediluted in blocking solution were applied for 60 min at room temperature or over night at 4°C. Antigen-antibody complexes were visualized using Alexa Fluor® 488 or 594 secondary antibodies (Invitrogen, Karlsruhe, Germany). Images were obtained by using a Nikon photomicroscope and processed with Adobe Photoshop 6.0 software.

*Isolation of total RNA:* Total RNA was isolated from cultured podocytes, Huvecs and PHH using RNeasy Kit (Qiagen, Hilden, Germany). RNase-free DNase-Set (Qiagen, Hilden, Germany) was used to remove potential sample contamination with genomic DNA. Quality and purity were assessed using capillary electrophoresis (Caliper Lab Chip system Agilent 2100 Bioanalyser, Agilent Technologies Inc., Waldbronn, Germany).

***Quantitative Reverse Transcription-PCR (qRT-PCR):*** Real-time quantitative PCR was performed using QuantiTect Probe RT-PCR Kit (Qiagen, Hilden, Germany). Each sample was assayed in tetraplicate. For relative quantification of gene expression the ΔΔCₜ method was used with GAPDH as a control. Amplification of target and house keeping gene were detected simultaneously by using differently fluorescent-labeled Taq Man probes which were obtained from Applera/Applied Biosystems (Foster City, USA). Amplification linearity of target and house keeping gene within multiplex RT-PCR was assessed by performing RT-PCR reaction with dilutions of templates. RT-PCR reaction and data acquisition was performed in iCycler-iQ-Thermocycler (Bio-Rad Laboratories GmbH, Munich, Germany). Relative gene expression was calculated as fold induction versus control samples. Minus values indicate fold down regulation in comparison with the expression level in control samples.

***Superarray-Technology:*** PCR Arrays are tools for analyzing the expression of a focused panel of genes. Each 96-well or includes SYBR Green-optimized primer assays for a thoroughly researched panel of relevant, pathway- or disease-focused genes (e.g. Transcriptional Factors, Cell Cycle, Stem Cells) and were obtained from SABiosciences (CA, USA).

The PCR array (RT² Profiler PCR Array System) performs gene expression analysis with real-time PCR sensitivity and the multi-gene profiling capability of a microarray. Simply mix your cDNA template (RT² First Strand Kit (C-03)) with the appropriate ready-to-use PCR master mix, aliquot equal volumes to each well of the same plate, and then run the real-time PCR cycling program. Plate templates and the real-time PCR cycling program (Instrument-Specific Setup Instructions & Protocol Files) are available using file http://www.sabiosciences.com/pcrarrayprotocolfiles.php.

The RT² First Strand Kit provides a rapid and convenient procedure for efficient first strand cDNA synthesis. The kit also contains an effective genomic DNA elimination step and a built-in External RNA Control. This all-in-one kit has been designed and optimized for real-time PCR-based gene expression analysis with SABiosciences' RT² Profiler™ PCR Arrays and RT² qPCR Primer Assays. The kit includes a proprietary procedure to effectively eliminate contaminating genomic DNA from RNA samples before reverse transcription. Random hexamers and oligo-dT prime reverse transcription in an unbiased manner and a reverse transcriptase synthesizes cDNA product with optimal yield and length. A built-in External RNA Control helps monitor reverse transcription efficiency and test for enzyme inhibitors contaminating your RNA samples when used together with RT² RNA QC PCR Array and RT² Profiler™ PCR Array. The magnesium and nucleotide concentrations and other buffer components are the most compatible with RT² SYBR Green qPCR Master Mixes when used in gene expression analysis with RT² Profiler™ PCR Arrays and RT² qPCR Primer Assays.

The RT² SYBR Green / Fluorescein qPCR master mix contains all of the reagents and buffers required for real-time polymerase chain reactions in the BioRad iCycler® and MyiQ®: real-time PCR buffer, a high-performance HotStart DNA Taq polymerase, nucleotides, SYBR® Green dye, and the Fluorescein reference dye needed to normalize the instruments' optics. Simply add the master mix to PCR tubes along with your template and primers. The chemically-modified and tightly controlled HotStart enzyme uniquely provides more accurate SYBR Green results by preventing the amplification of primer dimers and other non-specific products. This RT² qPCR master mix is best suited for real-time PCR applications using SYBR Green based detection on the BioRad iCycler® and MyiQ®.

### Sequences:

EF1alpha promoter (SEQ ID NO: 1) 
CGTGAGGCTCCGATGCCCGTCAGTGGGCAGAGCGCACATCGCCCACAGTCCC
CGAGAAGTTGGGGGGAGGGGTCGGCAATTGAACCGGTGCCTAGAGAAGGTGG
CGCGGGGTAAACTGGGAAAGTGATGTCGTGTACTGGCTCCGCCTTTTTCCCGA
GGGTGGGGGAGAACCGTATATAAGTGCAGTAGTCGCCGTGAACGTTCTTTTTC
GCAACGGGTTTGCCGCCAGAACACAGG
 LOX1 (SEQ ID NO: 2) GATCATAACTTCGTATAGCATACATTATACGAAGTTAT
LOX2 (SEQ ID NO: 3) TCGAATAACTTCGTATAGCATACATTATACGAAGTTAT

cDNA E6/E7 (SEQ ID NO: 4)

ATGTTTCAGGACCCACAGGAGCGACCCGGAAAGTTACCACAGTTATGCACAGA
GCTGCAAACAACTATACATGATATAATATTAGAATGTGTGTACTGCAAGCAACAG
TTACTGCGACGTGAGGTATATGACTTTGCTTTTCGGGATTTATGCATAGTATATA
GAGATGGGAATCCATATGCTGTATGTGATAAATGTTTAAAGTTTTATTCTAAAATT
AGTGAGTATAGACATTATTGTTATAGTGTGTATGGAACAACATTAGAACAGCAAT
ACAACAAACCGTTGTGTGATTTGTTAATTAGGTGTATTAACTGTCAAAAGCCACT
GTGTCCTGAAGAAAAGCAAAGACATCTGGACAAAAAGCAAAGATTCCATAATAT
AAGGGGTCGGTGGACCGGTCGATGTATGTCTTGTTGCAGATCATCAAGAACAC
GTAGAGAAACCCAGCTGTAATCATGCATGGAGATACACCTACATTGCATGAATA
TATGTTAGATTTGCAACCAGAGACAACTGATCTCTACTGTTATGAGCAATTAAAT
GACAGCTCAGAGGAGGAGGATGAAATAGATGGTCCAGCTGGACAAGCAGAACC
GGACAGAGCCCATTACAATATTGTAACCTTTTGTTGCAAGTGTGACTCTACGCTT
CGGTTGTGCGTACAAAGCACACACGTAGACATTCGTACTTTGGAAGACCTGTTA
ATGGGCACACTAGGAATTGTGTGCCCCATCTGTTCTCAGAAACCATAA

WPRE (SEQ ID NO: 5)

AATCAACCTCTGGATTACAAAATTTGTGAAAGATTGACTGGTATTCTTAACTATGT
TGCTCCTTTTACGCTATGTGGATACGCTGCTTTAATGCCTTTGTATCATGCTATT
GCTTCCCGTATGGCTTTCATTTTCTCCTCCTTGTATAAATCCTGGTTGCTGTCTC
TTTATGAGGAGTTGTGGCCCGTTGTCAGGCAACGTGGCGTGGTGTGCACTGTG
TTTGCTGACGCAACCCCCACTGGTTGGGGCATTGCCACCACCTGTCAGCTCCTT
TCCGGGACTTTCGCTTTCCCCCTCCCTATTGCCACGGCGGAACTCATCGCCGC
CTGCCTTGCCCGCTGCTGGACAGGGGCTCGGCTGTTGGGCACTGACAATTCCG
TGGTGTTGTCGGGGAAGCTGACGTCCTTTCCATGGCTGCTCGCCTGTGTTGCC
ACCTGGATTCTGCGCGGGACGTCCTTCTGCTACGTCCCTTCGGCCCTCAATCC
AGCGGACCTTCCTTCCCGCGGCCTGCTGCCGGCTCTGCGGCCTCTTCCGCGTC
TTCGCCTTCGCCCTCAGACGAGTCGGATCTCCCTTTGGGCCGCCTCCCCGCCT
G

Vector_pLV_EF1a-Lox1-E6E7-Lox2_WPRE (SEQ ID NO: 6)

CGCGTCGTGAGGCTCCGATGCCCGTCAGTGGGCAGAGCGCACATCGCCCACA
GTCCCCGAGAAGTTGGGGGGAGGGGTCGGCAATTGAACCGGTGCCTAGAGAA
GGTGGCGCGGGGTAAACTGGGAAAGTGATGTCGTGTACTGGCTCCGCCTTTTT
CCCGAGGGTGGGGGAGAACCGTATATAAGTGCAGTAGTCGCCGTGAACGTTCT
TTTTCGCAACGGGTTTGCCGCCAGAACACAGGATCATAACTTCGTATAGCATAC
ATTATACGAAGTTATGATCCGTTTAAACTTGCTGCGACGCGTACTAGTATGTTTC
AGGACCCACAGGAGCGACCCGGAAAGTTACCACAGTTATGCACAGAGCTGCAA
ACAACTATACATGATATAATATTAGAATGTGTGTACTGCAAGCAACAGTTACTGC
GACGTGAGGTATATGACTTTGCTTTTCGGGATTTATGCATAGTATATAGAGATGG
GAATCCATATGCTGTATGTGATAAATGTTTAAAGTTTTATTCTAAAATTAGTGAGT
ATAGACATTATTGTTATAGTGTGTATGGAACAACATTAGAACAGCAATACAACAA
ACCGTTGTGTGATTTGTTAATTAGGTGTATTAACTGTCAAAAGCCACTGTGTCCT
GAAGAAAAGCAAAGACATCTGGACAAAAAGCAAAGATTCCATAATATAAGGGGT
CGGTGGACCGGTCGATGTATGTCTTGTTGCAGATCATCAAGAACACGTAGAGAA
ACCCAGCTGTAATCATGCATGGAGATACACCTACATTGCATGAATATATGTTAGA
TTTGCAACCAGAGACAACTGATCTCTACTGTTATGAGCAATTAAATGACAGCTCA
GAGGAGGAGGATGAAATAGATGGTCCAGCTGGACAAGCAGAACCGGACAGAG
CCCATTACAATATTGTAACCTTTTGTTGCAAGTGTGACTCTACGCTTCGGTTGTG
CGTACAAAGCACACACGTAGACATTCGTACTTTGGAAGACCTGTTAATGGGCAC
ACTAGGAATTGTGTGCCCCATCTGTTCTCAGAAACCATAATCTAGCGTCGAATAA
CTTCGTATAGCATACATTATACGAAGTTATTCGACAATCAACCTCTGGATTACAA
AATTTGTGAAAGATTGACTGGTATTCTTAACTATGTTGCTCCTTTTACGCTATGTG
GATACGCTGCTTTAATGCCTTTGTATCATGCTATTGCTTCCCGTATGGCTTTCAT
TTTCTCCTCCTTGTATAAATCCTGGTTGCTGTCTCTTTATGAGGAGTTGTGGCCC
GTTGTCAGGCAACGTGGCGTGGTGTGCACTGTGTTTGCTGACGCAACCCCCAC
TGGTTGGGGCATTGCCACCACCTGTCAGCTCCTTTCCGGGACTTTCGCTTTCCC
CCTCCCTATTGCCACGGCGGAACTCATCGCCGCCTGCCTTGCCCGCTGCTGGA
CAGGGGCTCGGCTGTTGGGCACTGACAATTCCGTGGTGTTGTCGGGGAAGCTG
ACGTCCTTTCCATGGCTGCTCGCCTGTGTTGCCACCTGGATTCTGCGCGGGAC
GTCCTTCTGCTACGTCCCTTCGGCCCTCAATCCAGCGGACCTTCCTTCCCGCG
GCCTGCTGCCGGCTCTGCGGCCTCTTCCGCGTCTTCGCCTTCGCCCTCAGACG
AGTCGGATCTCCCTTTGGGCCGCCTCCCCGCCTGGAATTCGAGCTCGGTACCT
TTAAGACCAATGACTTACAAGGCAGCTGTAGATCTTAGCCACTTTTTAAAAGAAA
AGGGGGGACTGGAAGGGCTAATTCACTCCCAACGAAGACAAGATCTGCTTTTT
GCTTGTACTGGGTCTCTCTGGTTAGACCAGATCTGAGCCTGGGAGCTCTCTGG
CTAACTAGGGAACCCACTGCTTAAGCCTCAATAAAGCTTGCCTTGAGTGCTTCA
AGTAGTGTGTGCCCGTCTGTTGTGTGACTCTGGTAACTAGAGATCCCTCAGACC
CTTTTAGTCAGTGTGGAAAATCTCTAGCAGTAGTAGTTCATGTCATCTTATTATTC
AGTATTTATAACTTGCAAAGAAATGAATATCAGAGAGTGAGAGGAACTTGTTTAT
TGCAGCTTATAATGGTTACAAATAAAGCAATAGCATCACAAATTTCACAAATAAA
GCATTTTTTTCACTGCATTCTAGTTGTGGTTTGTCCAAACTCATCAATGTATCTTA
TCATGTCTGGCTCTAGCTATCCCGCCCCTAACTCCGCCCAGTTCCGCCCATTCT
CCGCCCCATGGCTGACTAATTTTTTTTATTTATGCAGAGGCCGAGGCCGCCTCG
GCCTCTGAGCTATTCCAGAAGTAGTGAGGAGGCTTTTTTGGAGGCCTAGGCTTT
TGCGTCGAGACGTACCCAATTCGCCCTATAGTGAGTCGTATTACGCGCGCTCAC
TGGCCGTCGTTTTACAACGTCGTGACTGGGAAAACCCTGGCGTTACCCAACTTA
ATCGCCTTGCAGCACATCCCCCTTTCGCCAGCTGGCGTAATAGCGAAGAGGCC
CGCACCGATCGCCCTTCCCAACAGTTGCGCAGCCTGAATGGCGAATGGCGCGA
CGCGCCCTGTAGCGGCGCATTAAGCGCGGCGGGTGTGGTGGTTACGCGCAGC
GTGACCGCTACACTTGCCAGCGCCCTAGCGCCCGCTCCTTTCGCTTTCTTCCCT

TCCTTTCTCGCCACGTTCGCCGGCTTTCCCCGTCAAGCTCTAAATCGGGGGCTC
CCTTTAGGGTTCCGATTTAGTGCTTTACGGCACCTCGACCCCAAAAAACTTGATT
AGGGTGATGGTTCACGTAGTGGGCCATCGCCCTGATAGACGGTTTTTCGCCCT
TTGACGTTGGAGTCCACGTTCTTTAATAGTGGACTCTTGTTCCAAACTGGAACAA
CACTCAACCCTATCTCGGTCTATTCTTTTGATTTATAAGGGATTTTGCCGATTTC
GGCCTATTGGTTAAAAAATGAGCTGATTTAACAAAAATTTAACGCGAATTTTAAC
AAAATATTAACGTTTACAATTTCCCAGGTGGCACTTTTCGGGGAAATGTGCGCG
GAACCCCTATTTGTTTATTTTTCTAAATACATTCAAATATGTATCCGCTCATGAGA
CAATAACCCTGATAAATGCTTCAATAATATTGAAAAAGGAAGAGTATGAGTATTC
AACATTTCCGTGTCGCCCTTATTCCCTTTTTTGCGGCATTTTGCCTTCCTGTTTTT
GCTCACCCAGAAACGCTGGTGAAAGTAAAAGATGCTGAAGATCAGTTGGGTGC
ACGAGTGGGTTACATCGAACTGGATCTCAACAGCGGTAAGATCCTTGAGAGTTT
TCGCCCCGAAGAACGTTTTCCAATGATGAGCACTTTTAAAGTTCTGCTATGTGG
CGCGGTATTATCCCGTATTGACGCCGGGCAAGAGCAACTCGGTCGCCGCATAC
ACTATTCTCAGAATGACTTGGTTGAGTACTCACCAGTCACAGAAAAGCATCTTAC
GGATGGCATGACAGTAAGAGAATTATGCAGTGCTGCCATAACCATGAGTGATAA
CACTGCGGCCAACTTACTTCTGACAACGATCGGAGGACCGAAGGAGCTAACCG
CTTTTTTGCACAACATGGGGGATCATGTAACTCGCCTTGATCGTTGGGAACCGG
AGCTGAATGAAGCCATACCAAACGACGAGCGTGACACCACGATGCCTGTAGCA
ATGGCAACAACGTTGCGCAAACTATTAACTGGCGAACTACTTACTCTAGCTTCC
CGGCAACAATTAATAGACTGGATGGAGGCGGATAAAGTTGCAGGACCACTTCT
GCGCTCGGCCCTTCCGGCTGGCTGGTTTATTGCTGATAAATCTGGAGCCGGTG
AGCGTGGGTCTCGCGGTATCATTGCAGCACTGGGGCCAGATGGTAAGCCCTCC
CGTATCGTAGTTATCTACACGACGGGGAGTCAGGCAACTATGGATGAACGAAAT
AGACAGATCGCTGAGATAGGTGCCTCACTGATTAAGCATTGGTAACTGTCAGAC
CAAGTTTACTCATATATACTTTAGATTGATTTAAAACTTCATTTTTAATTTAAAAGG
ATCTAGGTGAAGATCCTTTTTGATAATCTCATGACCAAAATCCCTTAACGTGAGT
TTTCGTTCCACTGAGCGTCAGACCCCGTAGAAAAGATCAAAGGATCTTCTTGAG
ATCCTTTTTTTCTGCGCGTAATCTGCTGCTTGCAAACAAAAAAACCACCGCTACC
AGCGGTGGTTTGTTTGCCGGATCAAGAGCTACCAACTCTTTTTCCGAAGGTAAC
TGGCTTCAGCAGAGCGCAGATACCAAATACTGTCCTTCTAGTGTAGCCGTAGTT
AGGCCACCACTTCAAGAACTCTGTAGCACCGCCTACATACCTCGCTCTGCTAAT
CCTGTTACCAGTGGCTGCTGCCAGTGGCGATAAGTCGTGTCTTACCGGGTTGG
ACTCAAGACGATAGTTACCGGATAAGGCGCAGCGGTCGGGCTGAACGGGGGG
TTCGTGCACACAGCCCAGCTTGGAGCGAACGACCTACACCGAACTGAGATACC
TACAGCGTGAGCTATGAGAAAGCGCCACGCTTCCCGAAGGGAGAAAGGCGGA
CAGGTATCCGGTAAGCGGCAGGGTCGGAACAGGAGAGCGCACGAGGGAGCTT
CCAGGGGGAAACGCCTGGTATCTTTATAGTCCTGTCGGGTTTCGCCACCTCTGA
CTTGAGCGTCGATTTTTGTGATGCTCGTCAGGGGGGCGGAGCCTATGGAAAAA
CGCCAGCAACGCGGCCTTTTTACGGTTCCTGGCCTTTTGCTGGCCTTTTGCTCA
CATGTTCTTTCCTGCGTTATCCCCTGATTCTGTGGATAACCGTATTACCGCCTTT
GAGTGAGCTGATACCGCTCGCCGCAGCCGAACGACCGAGCGCAGCGAGTCAG
TGAGCGAGGAAGCGGAAGAGCGCCCAATACGCAAACCGCCTCTCCCCGCGCG
TTGGCCGATTCATTAATGCAGCTGGCACGACAGGTTTCCCGACTGGAAAGCGG
GCAGTGAGCGCAACGCAATTAATGTGAGTTAGCTCACTCATTAGGCACCCCAG
GCTTTACACTTTATGCTTCCGGCTCGTATGTTGTGTGGAATTGTGAGCGGATAA
CAATTTCACACAGGAAACAGCTATGACCATGATTACGCCAAGCGCGCAATTAAC
CCTCACTAAAGGGAACAAAAGCTGGAGCTGCAAGCTTAATGTAGTCTTATGCAA
TACTCTTGTAGTCTTGCAACATGGTAACGATGAGTTAGCAACATGCCTTACAAG
GAGAGAAAAAGCACCGTGCATGCCGATTGGTGGAAGTAAGGTGGTACGATCGT

GCCTTATTAGGAAGGCAACAGACGGGTCTGACATGGATTGGACGAACCACTGA
ATTGCCGCATTGCAGAGATATTGTATTTAAGTGCCTAGCTCGATACAATAAACGG
GTCTCTCTGGTTAGACCAGATCTGAGCCTGGGAGCTCTCTGGCTAACTAGGGA
ACCCACTGCTTAAGCCTCAATAAAGCTTGCCTTGAGTGCTTCAAGTAGTGTGTG
CCCGTCTGTTGTGTGACTCTGGTAACTAGAGATCCCTCAGACCCTTTTAGTCAG
TGTGGAAAATCTCTAGCAGTGGCGCCCGAACAGGGACCTGAAAGCGAAAGGGA
AACCAGAGCTCTCTCGACGCAGGACTCGGCTTGCTGAAGCGCGCACGGCAAGA
GGCGAGGGGCGGCGACTGGTGAGTACGCCAAAAATTTTGACTAGCGGAGGCT
AGAAGGAGAGAGATGGGTGCGAGAGCGTCAGTATTAAGCGGGGGAGAATTAGA
TCGCGATGGGAAAAAATTCGGTTAAGGCCAGGGGGAAAGAAAAAATATAAATTA
AAACATATAGTATGGGCAAGCAGGGAGCTAGAACGATTCGCAGTTAATCCTGGC
CTGTTAGAAACATCAGAAGGCTGTAGACAAATACTGGGACAGCTACAACCATCC
CTTCAGACAGGATCAGAAGAACTTAGATCATTATATAATACAGTAGCAACCCTCT
ATTGTGTGCATCAAAGGATAGAGATAAAAGACACCAAGGAAGCTTTAGACAAGA
TAGAGGAAGAGCAAAACAAAAGTAAGACCACCGCACAGCAAGCGGCCGCTGAT
CTTCAGACCTGGAGGAGGAGATATGAGGGACAATTGGAGAAGTGAATTATATAA
ATATAAAGTAGTAAAAATTGAACCATTAGGAGTAGCACCCACCAAGGCAAAGAG
AAGAGTGGTGCAGAGAGAAAAAAGAGCAGTGGGAATAGGAGCTTTGTTCCTTG
GGTTCTTGGGAGCAGCAGGAAGCACTATGGGCGCAGCCTCAATGACGCTGACG
GTACAGGCCAGACAATTATTGTCTGGTATAGTGCAGCAGCAGAACAATTTGCTG
AGGGCTATTGAGGCGCAACAGCATCTGTTGCAACTCACAGTCTGGGGCATCAA
GCAGCTCCAGGCAAGAATCCTGGCTGTGGAAAGATACCTAAAGGATCAACAGC
TCCTGGGGATTTGGGGTTGCTCTGGAAAACTCATTTGCACCACTGCTGTGCCTT
GGAATGCTAGTTGGAGTAATAAATCTCTGGAACAGATTTGGAATCACACGACCT
GGATGGAGTGGGACAGAGAAATTAACAATTACACAAGCTTAATACACTCCTTAAT
TGAAGAATCGCAAAACCAGCAAGAAAAGAATGAACAAGAATTATTGGAATTAGAT
AAATGGGCAAGTTTGTGGAATTGGTTTAACATAACAAATTGGCTGTGGTATATAA
AATTATTCATAATGATAGTAGGAGGCTTGGTAGGTTTAAGAATAGTTTTTGCTGT
ACTTTCTATAGTGAATAGAGTTAGGCAGGGATATTCACCATTATCGTTTCAGACC
CACCTCCCAACCCCGAGGGGACCCGACAGGCCCGAAGGAATAGAAGAAGAAG
GTGGAGAGAGAGACAGAGACAGATCCATTCGATTAGTGAACGGATCTCGACGG
TATCGGTTAACTTTTAAAAGAAAAGGGGGGATTGGGGGGTACAGTGCAGGGGA
AAGAATAGTAGACATAATAGCAACAGACATACAAACTAAAGAATTACAAAAACAA
ATTACAAAAATTCAAAATTTTATCGATCACGAGACTAGCCTCGAGAAGCTTGAT

### RESULTS

### A Podocytes

Podocytes were isolated from the neonatal rat kidney glomeruli according to the protocol described by Mundel P et al. 1997. After isolation glomeruli were cultured in DMEM with 10% FCS on collagen-coated 24 well plates. 24 hours later podocytes precursors start to grow out from the attached glomeruli (Fig 2).

At this stage cells were transduced with the different lentiviral constructs and continued to be cultured for a prolonged period of time (2-4 weeks). Islands of the fast proliferating cells were isolated, expanded and characterized using RT PCR and immunostaining. Expression of the SV40 LTag has been controlled by immunostaining. 100% of the cells accumulate big amount of the SV40 LT Ag in the nuclei (Fig 3C) when in not transduced or GFP transduced cells no staining can be detected (data not shown). Transduction with the hTERT was insufficient to enhance the proliferation rate (though hTERT was overexpressed more than 500 fold according to the quantitative RT PCR data) and after several attempts has been abandoned. Transduction with the HPV E6/E7 alone was also not sufficient for the podocytes immortalization but when used in combination with the SV40 LT Ag increased significantly a number of the immortalized clones. After more than ten passages the cells were transduced with the lentiviral Cre recombinase. Three days later they were stained with the specific anti- SV40 LTag antibodies. Many cells showed a significant reduction or complete absence of the LT Ag, indicating that Cre excised all or majority of the integrated copies from the cellular genome (Fig 3D). Cells with the reduced (or negative) LT Ag stopped their proliferation, 3 increased in size and acquired a typical morphology of the podocytes (Fig 3 A and B). Efficiency of excision is relatively low; around 30-50%. Several highly specific genetic markers of podocytes were re-expressed in these cells, e.g., WT1, synaptopodin, though others, like nephrin and podocin were expressed at the extremely low levels (Fig 3 C-F and Table 1).

**Table 1: Expression of the podocyte-specific markers in immortalized cells**

| **SPECIFIC MARKER** | **fresh isolated podocytes [%]** | **LT Ag+E6E7 immortalized [%]** | **LT Ag + E6E7 (25d) + Cre (3d) [%]** |
|---|---|---|---|
| **NPHS1*** | 100 | 2 | 29 |
| **NPHS2*** | 100 | ND | ND |
| **Synaptopodin*** | 100 | 20 | 70 |
| **WT-1*** | 100 | 12 | 50 |
| **Size (Morphology)** | Cobblestone | Small oval | Arborized |
| **p53 (ICC)** | Low | High | Low |
| **SV40 LT Ag (ICC)** | Negative | High | Negative |

| | | | |
|---|---|---|---|
| ND...below limit of detection ICC... Immunocytochemistry *... Real Time PCR of the corresponding mRNA. For each gene the expression in the primary neonatal rat podocytes was accepted as 100%. | | | |

**Conclusion.** Podocytes can be successfully immortalized but should be reverted to a nonproliferating status by excision of the "immortogene" for further experimental use. Currently, an adenoviral Cre has been generated and will be tested with a purpose to increase an efficiency of excision. Without excision the immortalized cells proliferate extremely fast (doubling time 12-15 hours) and lose or express at a reduced level many specific markers of podocytes.

### B Primary human hepatocytes (PHH)

PHH were purchased from Lonza and cultured on collagen coated plates. The cells do not proliferate but can be sustained for 10-14 days in culture without visible signs of dedifferentiation. PHH transduced either with SV40 LT Ag or E6/E7 (but not with hTERT) and after several days start to proliferate, their size is reduced with time and they can be easily subcultured. We cultured these cells for more than 20 passages and their phenotype does not change though in comparison with the control hepatocytes they, definitely, loose partially their phenotype. In particular, expression of the hepatocytes-specific markers, like CRP and Haptoglobin is strongly reduced, especially in SV40 LT Ag though in E6/E7 immortalized cells, Haptoglobin expression is preserved and CRP expression is still relatively high - 10% in comparison with the primary cells (Table 2). Importantly, expression of the hepatocytesassociated transcription factors HNF4 alpha is reduced only two fold in E6/E7 transduced PHH and significantly more in SV40 LT Ag - 8 fold.

**Table 2: Expression of the PHH-specific genes and cell cycle regulating genes**

| | **Control PHH [%]** | **SV40 LT Ag immortalized [%]** | **E6E7 immortalized [%]** |
|---|---|---|---|
| **C-reactive** | **100** | 3 | 12 |
| **protein*** | **100** | 25 | 100 |
| **Haptoglobin*** | **100** | 3 | 25 |
| **LBP*** | **100** | 6 | 50 |
| **HNF4alpha*** | **100** | 15000 | 13000 |
| ***Cyclin A2**** | **100** | 24 | 16 |
| ***Cyclin D1**** | **100** | 3 | 6 |
| ***Cyclin D2**** | **100** | 21000 | 18600 |
| ***CDC2*/*CDK1**** | | | |
| **Size** | Big polyclonal | Small oval | Medium oval |
| **(Morphology)** | Low | High | Low |
| **p53 (ICC)** | Negative | High | Negative |
| **SV40 LT Ag (ICC)** | | | |

| | | | |
|---|---|---|---|
| ICC...Immunocytochemistry *... Real Time PCR of the corresponding mRNA. For each gene the expression in the primary human hepatocytes was accepted as 100%. | | | |

cDNA array analysis shows that immortalization caused by overexpression of SV40 LT Ag or E6/E7 viral early proteins strongly induces transcription of several genes known to be involved in control of cell cycle, namely E2F1 Transcription factor, cyclin A2, CDC2/CDK1- cyclin-dependent kinase 1 (Table 2). More detailed description of the transcriptome changes will be reported later.

SV40 LT Ag causes enormous accumulation of the nuclear p53 and E6/E7 - degradation and disappearance of p53 (Fig 4).

In both cases, p53 is completely inactivated, and loses it's transactivating and trans-repressive properties. In particular, expression of p21WAF1 (CIP1 or CDKN1A), an inhibitor of the CDK2 and one of the direct targets of p53, does not change (data not shown) in SV40 LT Ag-immortalized cells despite an accumulation of p53 in nuclei. On the other hand, mRNA expression of another CDK inhibitor, p16INK4 is strongly upregulated in the SV40 LT Ag and E6/E7-immortalized cells (data not shown). Potential mechanisms of this complicated phenomenon have been published but have no clear explanation (Pei and Xiong, 2005).

**Conclusion:** Primary human hepatocytes can be very efficiently immortalized with lentiviral mediated delivery of either SV40 LT Ag or human papilloma E6/E7 early proteins. Obtained cell lines grow extremely fast (doubling time is less than 20 hours) and still preserve many important markers of hepatocytes, especially in a case of the E6/E7-induced immortalization. These cells can be used for different biological purposes instead of primary non-dividing hepatocytes.

### C Primary Endothelial Cells

**HUVECs** (Human Umbilical Vein Endothelial Cells) were purchased from PromoCell and cultured in collagen-coated plates using Endothelial Cell-specific media containing FCS, ECGs, hEGF, hbFGF and HC-500. Subconfluent cells were transduced either with the SV40 LT Ag or E6/E7 lentiviral particles. Transduction of hTERT alone was not efficient (as in other tested cell types, as well). Higher proliferation rate was found in SV40 LT Ag and E6/E7-transduced cells and not in hTERT. FACS analysis demonstrated that both "immortogenes", LT Ag and E6/E7 cause a reduction (from 60 to 20%) of the G1/G0 fraction with a concomitant increase in the S-G2-M fractions. BrdU incorporation/staining (data not shown) confirmed FACS data. Surprisingly, despite much higher proliferation rate, the endothelium specific markers, CD31 (PECAM) and vWF were expressed to the same or even higher level in the immortalized HUVECs (see Fig 5 and Table 3).

**Table 3: Expression of the endothelial cell-specific markers and cell cycle regulating genes**

| | **Control HUVEC [%]** | **LT Ag immortalized [%]** | **E6E7 immortalized [%]** |
|---|---|---|---|
| **CD31*** | 100 | 42 | 350 |
| **vWF*** | 100 | 82 | 240 |
| ***Cyclin A2**** | 100 | 130 | 100 |
| ***Cyclin D1**** | 100 | 65 | 67 |
| ***Cyclin D2**** | 100 | 17 | 150 |
| ***CDC2*/*CDK1**** | 100 | 400 | 200 |
| **p53 (ICC)** | Low | High | Low |
| **SV40 LT Ag (ICC)** | Negative | High | Negative |

| | | | |
|---|---|---|---|
| ICC...Immunocytochemistry *... Real Time PCR of the corresponding mRNA. For each gene the expression in the primary human Huvec was accepted as 100%. | | | |

A moderate decrease of the endothelium specific NOS (eNOS) was detected in both, SV40 LT Ag and E6/E7 overexpressing HUVECs. Similar to other tested primary cells, SV40 LT Ag causes an enormous increase in the nuclear p53 protein and E6/E7 - decrease (data not shown).

Conclusion: Immortalized primary endothelial cells (HUVECs) preserve their main markers of differentiation though proliferate 3-5 times faster than parental cells. Morphologically, it was shown only cell size reduction and nuclear polymorphisms distinguish them from nonimmortalized cells. They can be used for the different types of experiments where an endothelial "background" is required.

**Efficiency of Immortalization.** Using lentiviral delivery system to overexpress early viral proteins SV40 LT Ag or HPV E6/E7 we were able to immortalize different primary human and rodent cells. Our primary goal was to immortalize primary rat kidney podocytes. Surprisingly, it turned to be much more difficult to transduce rodent cells with the lentiviruses in comparison with primary human cells. This probably can explain why we were unable to achieve immortalization of the rat kidney cells with the SV40 LT Ag or E6/E7 alone. But a combination of two lentiviral constructs (cells were transduced successively, first with SV40 LT Ag and two days later with the E6/E7) resulted in obtaining of several permanent cell lines from the isolated rat glomeruli. Fast proliferating cells partially lost differentiation markers and morphologically did not resemble podocytes. Therefore, it turned to be necessary to excise the "immortogenes" using Cre recombinase. Upon transduction with the lentiviral Cre, the cells stop to proliferate, increase in size and re-express the major podocyte-specific markers, including nephrin (NPHS1), synaptopodin, Wilms tumor 1 (WT1). Unfortunately, one of the principal podocytes-specific genes, podocin (NPHS2) did not re-appear in Cre-transduced cells. In general, we did observe that podocin was expressed only for a short time (up to 72 hours) in freshly isolated rat glomerular podocytes. Therefore, generated immortalized cells should be considered partially de-differentiated. What causes rapid disappearance of podocin expression (when many other podocyte-specific genes continue to be expressed) is not clear at present.

Initially, we planned to use for immortalization two additional genes, human catalytic subunit of Telomerase (hTERT) and thermo-sensitive (ts) SV40 Large T antigen mutant. Lentiviral particles containing these cDNA constructs were produced and overexpression of hTERT and SV40 LT Ag-ts has been confirmed in transduced cells but no immortalization has been achieved. hTERT has been tested in several human primary cells, including HUVECs, hepatocytes, smooth muscle cells, mesenchymal stem cells without any success. Therefore, it is necessary to conclude that hTERT alone is insufficient for immortalization, at least in mentioned above primary rodent and human cells.

Immortalization of primary human cells (mentioned above) has been successfully achieved with SV40 LT Ag and E6/E7 lentiviral constructs. The cells immortalized with SV40 LT Ag (independent of cells origin) proliferate much faster than E6/E7 expressing cells and show significant nuclear polymorphism with concomitant partial or complete loss of differentiation markers. Expression of E6/E7 results in activation of proliferation but causes only partial loss of differentiation and, therefore, represents more suitable system for generation of novel cell lines. Interestingly, in HUVECs, practically no loss of the differentiation status has been found (see Fig.6 and Table 4) and immortalized cells can be directly used without need for the "immortogene" excision.

**Mechanisms of Immortalization.** Large T-Antigen (LT Ag), a viral oncoprotein, is a major early gene product encoded by SV40. LT Ag and a member of the helicase family III (part of the AAA+ superfamily). This protein is critical for viral DNA replication (Li and Kelly 1984; Neuwald et al. 1999). SV40 LT Ag induces proliferation of quiescent cells, transform several cell types, and induce tumor formation in experimental animals (Fanning and Knippers 1992). Exact mechanism of immortalization/transformation caused by LT Ag is still not clear but majority of data indicate that it can overcome cell cycle checkpoints through interference with the function of several important cellular tumor suppressors including p53 (Srinivasan et al. 1997; Pipas 1998; DeCaprio 1999; Kim et al. 2001). In all tested primary human and rodent cells transduction with the lentiviruses coding for SV40 LT Ag leads to an enormous nuclear accumulation of p53 (Fig 3 and 4). On opposite, in primary cells transduced with HPV E6/E7 nuclear p53 disappears (Fig 4). Interestingly, several other viral oncoproteins (Adenovirus E1B, Hepatitis B virus protein X, Papilloma virus E6, Epstein-Barr virus EBNA-5, and the human T-cell lymphotropic virus tax protein) also target and inactivate p53 through enhanced proteosomal degradation. But SV40 LT Ag uses a principally different mechanism; stabilizing cellular p53 (increasing the half-life of the protein) while inhibiting its transactivation activity (Deppert et al. 1989; Pipas and Levine 2001). Mechanism of p53 stabilization and inactivation depends on a direct binding of SV40 LT Ag with p53. Recently, p53 has been co-crystallized with the Large T antigen. The structure shows an unexpected hexameric complex of LT Ag binding six p53 monomers. Bound SV40 LT prevents interaction of p53 DNA-binding domain with the p53 response elements in the promoters of p53-regulated genes. It is caused by the dramatic conformational changes at the DNA-binding area of p53, which is achieved partially through an unusual "methionine switch" within p53 (Lilyestrom W et al., 2006).

Inactivation of p53 in case of human papillomavirus (HPV) early protein E6 results from the enhanced degradation of the p53 through the ubiquitin pathway. E6 binds p53 protein through a cellular ubiquitin-ligase, the E6-associated protein (E6-AP), which recruits the ubiquitin complex of enzymes, ubiquitinating lysines on p53 and initiating its proteolysis.

HPV E7 inactivates the function of the retinoblastoma tumor suppressor protein pRb. E7 proteins are primarily localized in the nucleus, where they associate with retinoblastoma gene product pRb (pocket Rb) to facilitate progression into the S-phase of the cell cycle (Zur Hausen, 2002). In normal cells, pRb is hypophosphorylated in early G1 and bound to E2F transcription factors, forming complexes that function as the transcriptional repressors. Upon 9 phosphorylation the complexes dissociate, allowing E2F to act as a transcriptional activator. By associating with hypophosphorylated pRb, E7 prevents its binding with E2F, thereby promoting cell cycle progression. Additionally, the E7-induced ubiquitin-mediated pRb degradation appears to be essential in efficiently overcoming cell cycle arrest (Fehrmann & Laimins, 2003).

Therefore, a common mechanism involved in immortalization with SV40 and HPV is p53 and pRb inactivation. Very recent data indicate that p53 mediates not only transcriptional activation but repression, as well (Spurgers et al., 2006; Scian et al., 2008). Importantly, several p53-dependently repressed genes (cdc2, cdk4, cyclin 2A, cdc20, see Spurgers et al., 2006) were shown to be transactivated by E2F1-3 (Zhu et al., 2004). E2F1 upon dissociation from the pRb (either as a result of pRb phosphorylation or functional inactivation of pRb by viral proteins) activate its own transcription (positive feedback) and activate transcription of the target genes (cdc2, cdk4, cyclin 2A, cdc20) critical for G1/S and G2/M transition.

We analyzed expression of more than 200 genes involved in a control of proliferation and differentiation in the different cell types immortalized by either SV40 LT Ag or HPV E6/E7. In all cell types increased expression of E2F1, cdc2/cdk1 and cyclin A2 has been found, and in some cases (e.g., primary human hepatocytes) several hundreds fold (see Table 2). Cyclin A2 and cdc2/cdk1, as mentioned above, can be either transcriptionally repressed by p53 or transcriptionally activated by E2F1 (Fig 6). Therefore, we propose that SV40 and HPVmediated immortalization involves two critical events: first, inactivation of the p53 and pRb (shown previously by several groups) and second, release of transcriptionally active E2F proteins capable of stimulating transcription of E2F itself and its targets, cyclin A2, cyclin B and cdc2. As it was shown recently, cdc2/cdk1 is the only essential cell cycle Cdk in mammalian cells (Santamaria et al., 2007). These data indicate that Cdk1 can be considered as a critical regulator of immortalization and, probably carcinogenesis, as well caused by the double-stranded DNA HPV and SV40 viruses. Specific CDK1 inhibitors can be expected highly efficient against tumors with established HPV or SV40 etiology or with mutated p53 or Rb.

### REFERENCES

DeCaprio JA: The role of the J domain of SV40 large T in cellular transformation. Biologicals. 1999 Mar; 27(1):23-8.
Deppert W, Steinmayer T, Richter W: Cooperation of SV40 large T antigen and the cellular protein p53 in maintenance of cell transformation. Oncogene. 1989 Sep;4(9):1103-10.
Deppert W, Steinmayer T: Metabolic stabilization of p53 in SV40-transformed cells correlates with expression of the transformed phenotype but is independent from complex formation with SV40 large T antigen. Curr Top Microbiol Immunol. 1989; 44:77-83.
Fanning E, Knippers R: Structure and function of simian virus 40 large tumor antigen. Annu Rev Biochem. 1992; 61:55-85. Review.
Fehrmann F, Laimins LA: Human papillomaviruses: targeting differentiating epithelial cells for malignant transformation. Oncogene. 2003 Aug 11;22(33):5201-7. Review.
Li JJ, Kelly TJ: Simian virus 40 DNA replication in vitro. Proc Natl Acad Sci U S A. 1984 Nov; 81(22):6973-7.
Lilyestrom W, Klein MG, Zhang R, Joachimiak A, Chen XS: Crystal structure of SV40 large T-antigen bound to p53: interplay between a viral oncoprotein and a cellular tumor suppressor. Genes Dev. 2006 Sep 1; 20(17):2373-82.
Mundel P, Reiser J, Kriz W: Induction of differentiation in cultured rat and human podocytes. J Am Soc Nephrol 8: 697-705, 1997.
Neuwald AF, Aravind L, Spouge JL, Koonin EV: AAA+: A class of chaperone-like ATPases associated with the assembly, operation, and disassembly of protein complexes. Genome Res. 1999 Jan;9(1):27-43.
Pei XH, Xiong Y: Biochemical and cellular mechanisms of mammalian CDK inhibitors: a few unresolved issues. Oncogene. 2005 Apr 18; 24(17):2787-95. Review.
Pipas JM: Molecular chaperone function of the SV40 large T antigen. Dev Biol Stand.1998; 94:313-9. Review.
Pipas JM, Levine AJ: Role of T antigen interactions with p53 in tumorigenesis. Semin Cancer Biol. 2001 Feb; 11(1):23-30.
Santamaría D, Barrière C, Cerqueira A, Hunt S, Tardy C, Newton K, Cáceres JF, Dubus P, Malumbres M, Barbacid M: Cdk1 is sufficient to drive the mammalian cell cycle. Nature. 2007 Aug 16;448(7155):811-5.
Scian MJ, Carchman EH, Mohanraj L, Stagliano KE, Anderson MA, Deb D, Crane BM, Kiyono T, Windle B, Deb SP, Deb S: Wild-type p53 and p73 negatively regulate expression of proliferation related genes. Oncogene. 2008 Apr 17;27(18):2583-93. Epub 2007 Nov 5.
Srinivasan A, McClellan AJ, Vartikar J, Marks I, Cantalupo P, Li Y, Whyte P, Rundell K, Brodsky JL, Pipas JM: The amino-terminal transforming region of simian virus 40 large T and small t antigens functions as a J domain. Mol Cell Biol. 1997 Aug; 17(8):4761-73.
Spurgers KB, Gold DL, Coombes KR, Bohnenstiehl NL, Mullins B, Meyn RE, Logothetis CJ, McDonnell TJ: Identification of cell cycle regulatory genes as principal targets of p53- mediated transcriptional repression. J Biol Chem. 2006 Sep 1 ;281 (35):25134-42. Epub 2006 Jun 23.
Zhu W, Giangrande PH, Nevins JR: E2Fs link the control of G1/S and G2/M transcription. EMBO J. 2004 Nov 24;23(23):4615-26. Epub 2004 Oct 28
Zur Hausen H: Papillomaviruses and cancer: from basic studies to clinical application. Nat Rev Cancer. 2002 May; 2(5):342-50. Review.

## Claims

1. A method of producing a reversibly immortalized cell, the method comprising
a) providing a eukaryotic cell,
b) genetically modifying the cell of step a) to enable expression of one or more nucleic acid(s) coding for one or more immortalizing protein(s) in the cell; and
c) expressing the one or more nucleic acid(s) in the cell of step b), wherein the one or more immortalizing protein(s) include(s) human papillomavirus (HPV) E6/E7 or a functionally active variant thereof, optionally in combination with (i) SV40 Large T antigen (TAG) or a functionally active variant thereof and/or (ii) telomerase catalytic subunit (TERT) or a functionally active variant thereof.

2. A method of producing a reversibly immortalized cell, the method comprising
a) providing a eukaryotic cell,
b) genetically modifying the cell of step a) to enable expression of one or more nucleic acid(s) coding for one or more immortalizing protein(s) in the cell; and
c) expressing the one or more nucleic acid(s) in the cell of step b), wherein the at least one of the one or more immortalizing protein(s) is selected from the group consisting of human papillomavirus (HPV) E6/E7, SV40 Large T antigen and telomerase catalytic subunit (TERT), or a functionally active variant thereof,
and wherein expression of one or more of the nucleic acid(s) coding for one or more immortalizing proteins is controlled by an EF1alpha promoter.

3. The method of claim 1 or 2, wherein the method further comprises d) propagating the cell of step c).

4. The method of any of claims 1 to 3, wherein one or more nucleic acid(s) coding for immortalizing protein(s) is/are introduced by using one or more retroviral vector(s), particularly one or more lentiviral vector(s).

5. The method of any of claims 1 to 4, wherein the cell of step a) is (i) a mammalian cell, particularly a human, primate or rodent cell and/or (ii) a primary cell.

6. The method of any of claims 1 to 5, wherein the cell of step a) is a differentiated cell, particularly a endothelial cell, a cardiomyocyte, a smooth muscle cell, a hepatic cell, a podocyte or a kidney cell.

7. The method of any of claims 1 or 3 to 6, wherein expression of one or more of the nucleic acid(s) coding for one or more immortalizing proteins is controlled by an EF1alpha promoter.

8. The method of any of claims 1 to 7, wherein one or more of the nucleic acid(s) coding for one or more immortalizing proteins is/are flanked by loxP (locus of X-over P1) sites.

9. A cell obtainable by the method of any of claim 1 to 8, or a progeny thereof.

10. A eukaryotic cell comprising one or more nucleic acid(s) coding for one or more immortalizing protein(s), wherein the at least one of the one or more immortalizing protein(s) is selected from the group consisting of human papillomavirus (HPV) E6/E7, SV40 Large T antigen and telomerase catalytic subunit (TERT) and wherein expression of one or more of the nucleic acid(s) coding for one or more immortalizing protein(s) is controlled by an EF1alpha promoter.

11. The cell of claim 9 or 10, or a progeny thereof,
i) wherein the one or more nucleic acid(s) coding for immortalizing protein(s) is/are located on one or more retroviral vector(s), particularly one or more lentiviral vector(s);
ii) wherein the cell comprises a further genetic modification;
iii) wherein the cell is a mammalian cell, particularly a human, primate or rodent cell;
iv) wherein the cell is derived from a primary cell;
v) wherein the cell is derived from a differentiated cell, particularly an endothelial cell, a cardiomyocyte, a smooth muscle cell, a hepatic cell, a podocyte or a kidney cell;
vi) wherein expression of the nucleic acid(s) coding for the one or more immortalizing protein(s) is under the control of an EF1alpha promoter; and/or
vii) wherein one or more of the nucleic acid(s) coding for one or more immortalizing protein(s) is/are flanked by loxP (locus of X-over P1) sites.

12. A method of re-differentiating the cell of any of claims 9 to 11, or progeny thereof, the method comprising,
a) preventing expression of the one or more nucleic acid(s) coding for one or more immortalizing protein(s).

13. The method of claim 12, wherein one or more of the nucleic acid(s) coding for one or more immortalizing proteins is/are flanked by loxP (locus of X-over P1) sites and wherein the preventing is effected by Cre recombinase or a functionally active Cre variant.

14. The method of claim 12 or 13, wherein a Cre recombinase gene or a functionally active Cre variant gene is introduced into the cell using a retroviral vector, particularly a lentiviral vector.

15. A cell obtainable by the method of any of claims 12 to 14, or a progeny thereof.
